(19) Europäisches Patentamt
European Patent Office
Office européen des brevets

(11) **EP 2 705 140 B1**

(12) **EUROPÄISCHE PATENTSCHRIFT**

(45) Veröffentlichungstag und Bekanntmachung des Hinweises auf die Patenterteilung:
**21.03.2018  Patentblatt 2018/12**

(21) Anmeldenummer: **12730781.7**

(22) Anmeldetag: **07.05.2012**

(51) Int Cl.:
*C12N 1/20* *(2006.01)*   *C12R 1/225* *(2006.01)*
*A61K 35/74* *(2015.01)*   *A61K 8/99* *(2017.01)*
*A23L 33/135* *(2016.01)*  *A61Q 19/10* *(2006.01)*
*A61K 35/744* *(2015.01)*  *A61K 35/747* *(2015.01)*
*A61Q 17/00* *(2006.01)*   *C11D 3/38* *(2006.01)*
*C12Q 1/04* *(2006.01)*

(86) Internationale Anmeldenummer:
**PCT/DE2012/100129**

(87) Internationale Veröffentlichungsnummer:
**WO 2012/152270 (15.11.2012 Gazette 2012/46)**

(54) **NEUE MILCHSÄUREBAKTERIEN UND DIESE ENTHALTENDE ZUSAMMENSETZUNGEN**

NOVEL LACTIC ACID BACTERIA AND COMPOSITIONS CONTAINING THEM

NOUVELLES BACTÉRIES D'ACIDE LACTIQUE ET COMPOSITIONS LES CONTENANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.05.2011   DE 102011101134**

(43) Veröffentlichungstag der Anmeldung:
**12.03.2014   Patentblatt 2014/11**

(73) Patentinhaber: **Belano Medical AG
13355 Berlin (DE)**

(72) Erfinder:
• **LANG, Christine
  14057 Berlin (DE)**
• **RAAB, Andreas
  10589 Berlin (DE)**
• **GOLLETZ, Patrick
  12439 Berlin (DE)**

(74) Vertreter: **Hertin, Paul W. et al
Hertin und Partner
Rechts- und Patentanwälte PartG mbB
Kurfürstendamm 54/55
10707 Berlin (DE)**

(56) Entgegenhaltungen:
EP-A1- 1 634 948       WO-A1-2007/073709
WO-A1-2008/064893

• **N.A. Soleimani et al.: "Antagonistic activity of probiotic lactobacilli against Staphylococcus aureus isolated from bovine mastitis", African Journal of Microbiology Research, Bd. 4, Nr. 20 18. Oktober 2010 (2010-10-18), XP002683249, ISSN: 1996-0808 Gefunden im Internet: URL:http://www.academicjournals.org/ajmr/PDF/Pdf2010/18Oct/Soleimani%20et%20al.pdf [gefunden am 2012-09-09]**
• **LANG C ET AL: "Specific Lactobacillus/Mutans Streptococcus co-aggregation", JOURNAL OF DENTAL RESEARCH, INTERNATIONAL & AMERICAN ASSOCIATION FOR DENTAL RESEARCH, Bd. 89, Nr. 2, 1. Februar 2010 (2010-02-01), Seiten 175-179, XP009162445, ISSN: 1544-0591**
• **EVA M SÄDERLING ET AL: "Probiotic Lactobacilli Interfere with Biofilm Formation In Vitro", CURRENT MICROBIOLOGY, SPRINGER-VERLAG, NE, Bd. 62, Nr. 2, 11. September 2010 (2010-09-11), Seiten 618-622, XP019873919, ISSN: 1432-0991, DOI: 10.1007/S00284-010-9752-9**

EP 2 705 140 B1

- Jamalifar H. et al.: "Antimicrobial activity of different Lactobacillus species against multidrug resistant clinical isolates of Pseudomonas aeruginosa", Iranian Journal of Microbiology, Bd. 3, Nr. 1 1. März 2011 (2011-03-01), Seiten 21-25, XP002683250, Gefunden im Internet: URL:http://www.ncbi.nlm.nih.gov/pmc/articl es/PMC3279796/pdf/IJM-3-021.pdf [gefunden am 2012-09-11]

- JESSICA A. YOUNES ET AL: "Adhesion Forces and Coaggregation between Vaginal Staphylococci and Lactobacilli", PLOS ONE, Bd. 7, Nr. 5, 18. Mai 2012 (2012-05-18), Seiten e36917-e36917, XP55037146, ISSN: 1932-6203, DOI: 10.1371/journal.pone.0036917

**Beschreibung**

Die vorliegende Erfindung betrifft neue Milchsäurebakterien, oder Zelllysat, Derivat, Mutante oder Kombination davon, sowie Zusammensetzungen, die diese enthalten, insbesondere zur Verwendung als Probiotikum und/oder in der Körperhygiene und -therapie. Insbesondere betrifft die vorliegende Erfindung die Verwendung der neuen Milchsäurebakterien bzw. der dieser enthaltenden Zusammensetzungen zur Behandlung und/oder Prophylaxe allen Erkrankungen, welche durch *Staphylococcus aureus* oder *Pseudomonas aeruginosa* hervorgerufen werden können.

Des Weiteren stellt die hier beschriebene Entwicklung ein innovatives biologisches Produktes in Form von GRAS-Mikroorganismen, Milchsäurebakterien, das als spezifisch wirkendes antimikrobielles Additiv für die lokale Behandlung von Hautinfektionen und die Beschleunigung der Heilung von chronischen Wunden eingesetzt werden kann.

Ferner betrifft die vorliegende Erfindung die Verwendung des erfindungsgemäßen Mikroorganismus, oder Zelllysat, Derivat, Mutante oder Kombination davon in Zusammensetzungen oder pharmazeutischen Produkten oder kosmetischen Produkten oder medizintechnischen Produkten, sowie auch als Haut- oder Flächendesinfektionsmittel.

## HINTERGRUND

Die Hauptfunktion der Haut ist der Schutz des darunter liegenden Gewebes vor der äußeren Umgebung. So verhindert sie unter anderem das Eindringen von pathogenen Mikroorganismen in den Körper. Natürlicherweise sind Haut und Schleimhäute von den verschiedensten Mikroorganismen besiedelt, die oftmals als Kommensalen in einer relativ stabilen Zusammensetzung auf der Oberfläche leben und die Schutzfunktion der Haut unterstützen. Im Idealfall dominieren Bakterien mit einer positiven gesundheitlichen Wirkung über gleichfalls vorkommende, schädliche Keime. Gerät dieses System aus dem Gleichgewicht, sind negative Auswirkungen auf die Gesundheit und das Wohlbefinden vorprogrammiert.

Pathogene Mikroorganismen besitzen die Fähigkeit, sich spezifisch durch Bindeproteine an Strukturen der Epidermis anzuheften. Für den pathogenen Stamm *Staphylococcus aureus* ist beispielsweise das Vorhandensein von Adhäsinen bekannt, mit denen sich der Organismus an Fibronectin-Strukturen anheften kann (Bingham R. J. et al. 2008, O'Neill E. et al. 2008). Pathogene Mikroorgansimen haben in der Regel ein höheres Potential, sich an den Wirt anzuheften, wodurch die erhöhte Virulenz zu erklären ist. Das Vorhandensein von kleinsten Läsionen oder sonstigen Schädigungen der obersten Hautschichten erhöht die Gefahr der Besiedlung mit pathogenen Mikroorganismen.

Ferner können bakterielle Infektionen der Wundoberflächen insbesondere bei der Heilung von Wunden zu Komplikationen führen. Es besteht die Gefahr, dass zunächst akute Wunden nicht heilen und zu chronischen Wunden werden. Die Mikroflora dieser chronischen Wunden ist sehr komplex und es ist bekannt, dass eine Vielzahl von Mikroorganismen den Heilungsprozess von diesen Wunden beeinträchtigen kann (Davies et al. 2004, Kirketerp-Møller et al. 2008).

Als Hauptpathogene in Wunden sind aerobe Bakterien, wie z.B. *Pseudomonas aeruginosa* und *Staphylococcus aureus* identifiziert worden. Normalerweise dient die Entzündungsphase der Wundheilung zur Bekämpfung potentiell pathogener Mikroorganismen und zur Zellregeneration. Allerdings treten schlecht oder nicht heilende Wunden oft bei bereits immunsupprimierten Patienten auf, die eine verminderte Entzündungsantwort aufweisen. Diese geschwächte Immunantwort kann die primären Wundbakterien nicht mehr effektiv abwehren, so dass die Bakterien in die Wunde eindringen und Gemeinschaften bilden, die als Biofilme organisiert sind (James et al. 2008).

Die Biofilme sind nicht nur resistent gegen das Abwehrsystem des Wirtes sondern auch planktonische Zellen oder Mikrokolonien (Fux et al. 2005, Sheldon 2005). Durch das beeinträchtigte Immunsystem hält der Biofilm den Heilungsprozess der Wunde in der Entzündungsphase mit der Folge, dass erhöhte Konzentrationen an Matrix Metalloproteinasen wie z.B. Elastase, Plasmin und Thrombin auftreten, die wiederum Wachstumsfaktoren und ihre Rezeptoren abbauen, die essentiell für die Heilung sind (Mast und Schultz, 1996).

Ferner führen die erhöhten Konzentrationen an freien Sauerstoffradikalen und inflammatorischen Cytokinen zu starken Schädigungen der Wirtszellen (James et al. 2003, Moseley et al. 2004).

Im Stand der Technik sind therapeutische Mittel beschrieben, die in der Lage sind, den Biofilm zu eliminieren und damit die Ursachen der chronischen bzw. schlecht heilenden Wunden zu bekämpfen.

## STAND DER TECHNIK

Die Behandlung von Wundinfektionen wird nach derzeitigem Stand durch komplexe Antibiotika-Therapien durchgeführt. Diese führen nicht in jedem Fall zur Heilung, da die benannten Wundkeime aufgrund ihrer Anpassungsfähigkeit und Resistenzmechanismen einschließlich ihrer Fähigkeit zur Biofilmbildung auf eine solche Therapie nicht ansprechen. Für eine prophylaktische Verhinderung von Hautschädigungen, vor allem bei immunsupprimierten Patienten, z.B. mit atopischer Dermatitis, Ekzemen, sebrorrhoische Dermatitis werden hautschonende Pflegeprodukte, wie pH-optimierte Duschgele, Waschlotionen, Duschöle und Körperlotionen angeboten. Deren dermatologisch getestete Inhaltsstoffe, sind meist frei von Emulgatoren und Konservierungsstoffen und sollen die Beschaffenheit empfindlicher Haut schützen.

Das Hautpathogen *S. aureus* ist ein Krankheitserreger, welcher auch ohne Nahrung bis zu sieben Monate überleben kann. Er überdauert auf Wäsche oder Türklinken, Lichtschaltern, auf dem Fußboden oder auf der Bettkante (Julia Bidder 2010). Bei gesunden Menschen verursacht er keine Beschwerden. Ist das Immunsystem geschwächt, wie bei einer Wundinfektion vermehrt sich der Keim und verursacht schlecht heilende Entzündungen, Hautgeschwüre, Furunkel, Lungenentzündungen, Harnweginfektionen und lebensgefährliche Blutvergiftungen, Augeninfektionen, Mittelohrentzündungen. Die Entzündungen können praktisch auf jedes Organ überspringen. Bestimmte Faktoren können eine Infektion mit *Staphylococcus aureus* begünstigen, wie z.B. eine geschwächte Immunabwehr, Diabetes mellitus, bestehende Schäden an der Haut (Schuppenflechte oder Neurodermitis), Hautverletzungen (z.B. durch Unfälle, Operationen, Katheter), alte Menschen mit Dekubitus und bettlägerige Menschen, übergewichtige Menschen, deren Eigenhaut feuchte Reservoire für die Keimentwicklung bietet.

Bei methicillin-resistenten - *S. aureus* - Stämmen (MRSA) wirkt das Antibiotikum Methicillin nicht mehr. In der Praxis bedeutet dies, dass dieser Stamm multiresistent gegen drei oder mehr Antibiotika ist. Gegen diese Stämme helfen nur sogenannte Reserveantibiotika. Deutsche Kliniken beziffern die Mehrkosten für die Behandlung eines MRSA-Patienten auf 1600 bis 4300 Euro pro Tag (Julia Bidder 2010).

Ein weiteres Hautpathogen stellt *Pseudomonas aeruginosa* dar. Auch dieser Stamm ist häufig als Krankenhauskeim zu finden und weist durch seinen Stoffwechsel und seine Zellmembranstruktur Mehrfachresistenzen gegenüber Antibiotika auf. Mit ca. 10 % aller Krankenhausinfektionen gehört *P. aeruginosa* zu den in Deutschland am häufigsten auftretenden Krankenhauskeimen. Das Spektrum an Krankheiten, welche durch diese Bakterien verursacht werden, ist umfangreich. Auslöser dafür sind zum einen die Fähigkeit zur Hämolyse und zum anderen Pathogenitätsfaktoren wie das Exotoxin A (ADP-Ribosyltransferase) sowie die Cytotoxine Exoenzym S und Exoenzym U, die das Bakterium produziert. Das häufigste Erscheinungsbild sind Pneumonien bei zystischer Fibrose, die vor allem bei immunsupprimierten und AIDS-Patienten besonders schwerwiegend sind. Harnwegsinfekte, Enterokolitis, Meningitis, Otitis externa ("swimmer's ear"), Infektionen auf Brandwunden oder Keratitis bei Kontaktlinsenträgern können ebenfalls ausgelöst werden.

Milchsäurebakterien werden im Allgemeinen als probiotische Bakterien für den Schutz vor Magen-Darm-Erkrankungen eingesetzt, die durch Krankheitserreger ausgelöst werden, da sie neben Milchsäure auch häufig anti-bakterielle Substanzen produzieren. Die Milchsäurebakterien (Lactobacillales, Lactobazillen oder Sauermilchbakterien) bilden eine Ordnung von grampositiven, stets anaeroben aber meist aerotoleranten Bakterien, die sich dadurch auszeichnen, dass sie Zucker zu Milchsäure abbauen (Milchsäuregärung). Die Ordnung der Lactobacillales umfasst die Familien Lactobacillaceae, Aerococcaceae, Carnobacteriaceae, Enterococcaceae, Leuconostocaceae und Streptococcaceae. Früher wurde die Art *Bifidobacterium bifidum* zu den Laktobazillen gestellt (*Lactobacillus bifidum*)*,* wobei es nach heutigem Stand phylogenetisch mit der Ordnung nicht in näherer Verbindung steht. Den Stoffwechsel betreffend wird es aber weiterhin als Milchsäurebakterium behandelt. Milchsäurebakterien sind außerdem für die Lebensmittelindustrie von hoher Wichtigkeit, da sie für die Herstellung von Milchprodukten genutzt werden, aber auch als Schädlinge (z. B. in der Bierbrauerei) auftreten können. Die Milchsäurebakterien werden als apathogen bezeichnet.

Im Stand der Technik (z. B. WO 2010/130563 ist die Verwendung von probiotischen Bakterien für Geschirrspülmittel, wodurch die negativen Folgen des Handgeschirrspülens für die Haut verringert werden. Außerdem stellt sich ein pflegender Effekt für die Haut ein.

Der Einsatz derartiger Mikroorganismen in kosmetischen Hautbehandlungsmitteln ist bereits bekannt. So wird etwa in US 6790434 die Verwendung solcher Mikroorganismen in kosmetischen Hautbehandlungsmitteln in Verbindung mit einem pflanzlichen Extrakt aus der extrazellulären Matrix beschrieben, um hierdurch der durch UV-Strahlung bedingten Hautschädigung entgegen zu wirken. Jedoch wird der Einsatz dieser Mikroorganismen in Wasch- und Reinigungsmitteln nicht offenbart.

Weiterhin ist der Einsatz bestimmter Bacillus-Arten in Sanitärreinigungsmitteln bekannt. So wird in WO 97/25865 der Einsatz von Bacillus-Arten in Sanitärreinigungsmitteln beschrieben, die die Vermehrung von Pathogenen vermeiden und organischen Schmutz abbauen sollen. Der Einsatz von Mikroorganismen mit einem Benefit-Effekt auf die Haut wird hier jedoch nicht offenbart.

In der Druckschrift Soleimani et al (Antagonistic activity of probiotic lactobacilli against Staphylococcus aureus isolated from bovine mastitis, African Journal of Microbiology Research, Bd. 4, Nr. 20, 18. Oktober 2010) wird die Kongregation von verschiedenen Laktobazillus-Stämmen mit Staphylococcus aureus beschrieben.

Im Stand der Technik sind außerdem Zusammensetzungen und Verwendungen von Milchsäurebakterien bekannt, welche das Wachstum von pathogenen Mikroorganismen, insbesondere Staphylococcus aureus auf der Haut hemmen (WO 2008/064893 A1).

Eine orale Darreichungsform von probiotischen Bakterien ist mitunter in der WO 2005/117921 offenbart. Hierbei weist die Darreichungsform wenigstens eine Gattung von probiotischen Mikroorganismen auf, wobei die Darreichungsform und/oder die Bakterien mit einem Celluloseether enthaltenen Überzug versehen ist.

Aufgabe der Erfindung ist es, ein Mittel oder Zusammensetzung zur akuten und prophylaktischen Behandlung von Wundinfektionen und Hautkrankheiten -bzw. -irritationen bereitzustellen, das nicht die Nachteile oder Mängel des Standes der Technik aufweist.

## DARSTELLUNG DER ERFINDUNG

Gelöst wird die Aufgabe durch die unabhängigen Ansprüche. Bevorzugte Ausführungsformen ergeben sich aus den Unteransprüchen.

Mitunter betrifft die Erfindung einen Mikroorganismus, der Ordnung Milchsäurebakterien, wobei der Mikroorganismus ausgewählt ist aus der Gruppe umfassend folgende bei der Deutschen Sammlung für Mikroorganismen und Zellkulturen hinterleg-ten als DSM 25906, DSM 25907, DSM 25908, DSM 25909, DSM 25910, DSM 25911, DSM 25912, DSM 25913, DSM 25914 und DSMZ 25915 nummerierten Mikroorganismenoder ein Derivat, Mutante, Zelllysatoder Kombination davon, wobei der Mikroorganismus, oder Zelllysat, Derivat, Mutante oder Kombination davon mit mindestens einem pathogenen Mikroorganismus koaggregieren kann, wobei der pathogene Mikroorganismus ausgewählt ist aus der Gruppe umfassend Staphylococcus aureus oder Pseudomonas aeruginosa. Es war völlig überraschend, dass neue Milchsäurebakterien bereitgestellt werden können, welche insbesondere infektiöse Bakterienstämme aggregieren, insbesondere koaggregieren und somit die örtliche Konzentration der Keime reduzieren, in ihrem Wachstum hemmen oder sogar abtöten können und/oder die Biofilmbildung verhindern. Dies stellt eine Abkehr vom Stand der Technik dar, mit entscheidenden Vorteilen gegenüber herkömmlichen Behandlungsmöglichkeiten des Standes der Technik, da keine Antibiotikabehandlung notwendig ist, sondern die apathogenen Milchsäurebakterien verwendet werden. Zudem stellt die Produktion von spezifischen Milchsäurebakterien im Vergleich zur Antibiotikaproduktion und folgenden Behandlungskosten eine preiswertere Alternative dar. Die Behandlung mit Milchsäurebakterien, welche spezifisch gegen diese Keime gerichtet ist und keine weiteren Resistenzen der Keime auslöst, ist ein einzigartiger Ansatz im Kampf gegen die pathogenen Keime.

Koaggregation beschreibt im Sinne der Erfindung insbesondere eine Haftung oder Bindung genetisch unterschiedlicher Bakterienarten aneinander, während man bei Koadhäsion insbesondere von der Haftung oder Bindung genetisch identischer Bakterienarten spricht. Diesbezüglich war umso überraschender, dass Milchsäurebakterien an die pathogenen Bakterien binden können, wobei durch die insbesondere spezifische Bindung eine Koaggregation entsteht.

Insbesondere betrifft die Erfindung einen zuvor genannten Mikroorganismus oder Zelllysat, Derivat, Mutante oder Kombination davon, wobei die Fähigkeit zur Koaggregation des zumindest einen pathogenen Mikroorganismus auch nach einer biologischen, chemischen oder physikalischen Behandlung besteht. Bei dem bevorzugten Mikroorganismus oder Zelllysat,, Derivat, Mutante oder Kombination davon besteht die Fähigkeit zur Koaggregation des zumindest einen pathogenen Mikroorganismus bevorzugt auch noch bei einem pH-Wert von zwischen ca. 3 bis ca. 8. In einer bevorzugten Ausführungsform besitzt der Mikroorganismus oder Zelllysat, Derivat, Mutante oder Kombination davon insbesondere die Fähigkeit zur Hemmung einer Bildung eines Biofilms des zumindest einen pathogenen Mikroorganismus.

In einem weiteren Aspekt betrifft die Erfindung eine Zusammensetzung umfassend mindestens einen der genannten Mikroorganismen oder deren Zelllysat, Derivat, Mutante oder Kombination. Der Begriff Zusammensetzung kann im Sinne der Erfindung synonym zu dem Begriff Formulierung verwendet werden. Die bevorzugte Zusammensetzung kann zudem einen Träger oder Exzipienten, ausgewählt aus der Gruppe umfassend kosmetisch verträgliche Träger oder Exzipienten, pharmazeutisch verträgliche Träger oder Exzipienten oder dermatologisch verträgliche Träger oder Exzipienten umfassen.

Die Zusammensetzung kann bevorzugt als Puder, in Stiftform, als Aerosolspray, Pumpspray, Creme, Dispersion, Emulsion, Schaum, Salbe, Spray, Aerosol, Pulver, Stift, Tücher, Lotion, Suspension, Lösung, Gel oder auf einem Substrat vorliegen. Insbesondere ist es bevorzugt, dass die Zusammensetzung als Hautcreme, Hautwaschlotion oder Hautsalbe vorliegt.

In einer bevorzugten Ausführungsform kann die Zusammensetzung in fester, flüssiger, viskoser Form oder als Aerosol vorliegen. Ferner kann insbesondere die bevorzugte Hautcreme, Hautwaschlotion oder Hautsalbe in fester, flüssiger, viskoser Form oder als Aerosol vorliegen.

Die bevorzugte Zusammensetzung kann zudem bevorzugt Probiotika, Antiseptika oder andere antibakteriell wirksame Substanzen umfassen, wobei es sich bei einer bevorzugten Zusammensetzung um eine pharmazeutische, tiermedizinische, kosmetische oder Nahrungsmittelzusammensetzung handelt.

In einer weiteren bevorzugten Ausführungsform umfasst die Zusammensetzung bevorzugt weiterhin Buildersubstanzen, oberflächenaktive Tenside, Enzyme, organische und/oder anorganische Persauerstoffverbindungen, Persauerstoff-Aktivatoren, wassermischbare organische Lösungsmittel, Sequestrierungsmittel, Elektrolyte, pH-Regulatoren, Verdicker, Soil release-Wirkstoffe, optische Aufheller, Vergrauungsinhibitoren, Farbübertragungsinhibitoren, Schaumregulatoren und/oder Farbstoffe.

Weiterhin kann es bevorzugt sein, dass die Zusammensetzung zumindest einen Stoff umfasst, ausgewählt aus den Gruppen

a. Wirkstoffe, die den Zustand der Haut positiv beeinflussen, insbesondere Wirkstoffe zur positiven Beeinflussung der Altershaut, insbesondere in Kombination mit Biochinone, insbesondere Ubichinon Q10, Kreatin, Kreatinin, Carnitin, Biotin, Isoflavon, Cardiolipin, Liponsäure, Anti Freezing Proteine, Arctiin, Hopfen- und Hopfen-Malz-Extrakte,

b. fördernde Mitteln zur Restrukturierung des Bindegewebes, insbesondere Isoflavonoide,

c. Wirkstoffen zur Unterstützung der Hautfunktionen bei trockener Haut, insbesondere Vitamin C, Biotin, Carnitin, Kreatin, Propionsäure, Grüntee-Extrakte, Eucalyptusöl, Harnstoff und Mineralsalze, insbesondere NaCl, Meeresmineralien sowie Osmolyte,

d. Wirkstoffen zur Linderung und/oder positiven Beeinflussung von irritativen Hautzuständen, insbesondere Sericoside, verschiedene Extrakte des Süssholzes, Licochalcone, insbesondere Licochalcone A, Silymarin, Silyphos und/oder Dexpanthenol.

Es ist bevorzugt, dass der Mikroorganismus inaktiviert, lebend oder nicht-lebend in der Zusammensetzung vorliegt. Weiterhin kann der Mikroorganismus bevorzugt in verkapselter, sprühgetrockneter und/oder lyophilisierter Form in der Zusammensetzung vorliegen. Bevorzugt ist zudem, dass der der Mikroorganismus insbesondere in Form eines Zelllysates in der Zusammensetzung vorliegt. In einer bevorzugten Ausführungsform liegt der Mikroorganismus in der Zusammensetzung insbesondere in einer Menge mit einem Masseanteil von 0,001 Gewichtsprozent bis 10 Gewichtsprozent, bevorzugt bis 0,005 Gewichtsprozent bis 5 Gewichtsprozent, besonders bevorzugt 0,01 Gewichtsprozent bis 3 Gewichtsprozent vor.

In einem weiteren Aspekt betrifft die Erfindung die Verwendung der Zusammensetzung bevorzugt zur Herstellung eines Arzneimittels, Medizinproduktes oder Kosmetikums zur Behandlung oder Prophylaxe von Hauterkrankungen, insbesondere Staphylococcal scalded skin syndrome, Impetigo contagiosa, Folliculitis superficialis, Impetiginisation, Hautabszesse, Furunkel, Karbunkel, Eiterbeule, Phlegmone, trockene Haut, juckende Haut, gerötete Haut, irritierte Haut, stark fettende Haut, Akne, diabetischer Fuß, Dekubitus, Neurodermitis, Akute Lymphadenitis, Pilonidalzysten, Pilonidalfistel, Pilonidalsinus, Steißbeinfistel, Steißbeinzyste, lokale Infektionen der Haut und der Unterhaut, Pyodermie, Dermatitis purulenta, Dermatitis septica, Dermatitis suppurativa, Dermatitis und Ekzeme, atopisches Ekzem, seborrhoisches Ekzem, Windeldermatitis, allergische Kontaktdermatitis, seborrhoisches Dermatitis, exfoliative Dermatitis, toxische Kontaktdermatitis, Lichen simplex chronicus, Prurigo, Pruritus sowie sonstige Dermatitis, papulosquamöse Hautkrankheiten, Psoriasis, Parapsoriasis, Krankheiten der Hautanhangsgebilde, narbige Alopezie, Folliculitis decalvans, sowie sonstige Erkrankungen der Haut und Unterhaut, Ulcus cruris, Hautverletzungen, Schürfwunden, Wunden, auch nach Unfällen oder Operationen.

Die Zusammensetzung kann in einer bevorzugten Ausgestaltung zur Herstellung eines Reinigungsmittels oder Desinfektionsmittels für die Behandlung von Oberflächen verwendet werden. Weiterhin kann die Zusammensetzung vorteilhafterweise zur Herstellung eines Produktes verwendet werden, das im Bereich der Körperhygiene, Medizinprodukte und Prophylaxe eingesetzt wird.

Bevorzugt wird die Zusammensetzung zur Herstellung einer Lotion, Schüttelmixtur, Puder, Hydrogel, Creme, Cresa, Salbe, Fettsalbe oder Paste zur Aufbringung auf eine Hautfläche verwendet.

Es hat vorteilhafterweise herausgestellt, dass die Zusammensetzung bevorzugt zur Herstellung eines spezifisch wirkendes antimikrobielles Additiv für die lokale Behandlung von Hautinfektionen und die Beschleunigung der Heilung von chronischen Wunden verwendet werden kann. Die Verwendung der Zusammensetzung kann bevorzugt prophylaktisch oder kurativ erfolgen. Außerdem kann die Zusammensetzung bevorzugt topisch appliziert werden. In einem weiteren Aspekt betrifft die Erfindung ein Kit zur Hygienebehandlung, umfassend Mikroorganismen oder die Zusammensetzung und körperhygienische Vorrichtungen oder Geräte, Spülungen und/oder Pasten.

In einer bevorzugten Anwendungsform kann die Erfindung als Spray oder Waschlösung für Tiere, insbesondere für Hunde, Pferde, Katzen und Nagetiere (Hasen, Kaninchen, Hamster, Meerschweinchen) und Nutztiere, wie Huhn, Schwein und Rind als antimikrobiell wirkendes Additiv eingesetzt werden, um die Keimbelastung von Haut, Fell und Federn signifikant zu reduzieren.

Die vorliegende Erfindung betrifft bevorzugt neue Milchsäurebakterien, Zelllysat Mutanten oder Derivate davon, sowie Zusammensetzungen, die diese enthalten, insbesondere zur Verwendung zur Behandlung oder Prophylaxe von Säuglingen, Kleinkindern, Kindern, gesunden Menschen, alten Menschen, immunsupprimierten Menschen, Menschen mit krankhaften Hautveränderungen (insbesondere Staphylococcal scalded skin syndrome, Impetigo contagiosa, Folliculitis superficialis, Impetiginisation, Hautabszesse, Furunkel (Furunkulose), Karbunkel (Eiterbeule), Phlegmone, trockene Haut, juckende Haut, gerötete Haut, irritierte Haut, stark fettende Haut, Akne, diabetischer Fuß, Dekubitus, Neurodermitis, Akute Lymphadenitis, Pilonidalzysten (inkl. Pilonidalfistel, Pilonidalsinus, Steißbeinfistel, Steißbeinzyste), sonstige lokale Infektionen der Haut und der Unterhaut (z.B. Pyodermie, Dermatitis purulenta, Dermatitis septica, Dermatitis suppurativa); des Weiteren Dermatitis und Ekzeme (z.B. atopisches Ekzem, seborrhoisches Ekzem, Windeldermatitis, allergische Kontaktdermatitis, seborrhoisches Dermatitis, exfoliative Dermatitis, toxische Kontaktdermatitis, Lichen simplex chronicus, Prurigo, Pruritus sowie sonstige Dermatitis; weiterhin papulosquamöse Hautkrankheiten (Psoriasis, Parapsoriasis), Krankheiten der Hautanhangsgebilde (z.B. narbige Alopezie inkl. Folliculitis decalvans), sowie sonstige Erkrankungen der Haut und Unterhaut (z.B. Ulcus cruris), Personen mit bestehenden Schäden an der Haut (z.B. trockene Haut), Hautverletzungen (z.B. Schürfwunden, Wunden, auch nach Unfällen oder Operationen) oder von Nutz- und Haustieren eingesetzt werden kann.

Die bevorzugten Mikroorganismen, nämlich die Mikroorganismen, der zur Ordnung der Milchsäurebakterien ausgewählt aus der Gruppe umfassend die Stämme DSM 25906, DSM 25907, DSM 25908, DSM 25909, DSM 25910, DSM 25911, DSM 25912, DSM 25913, DSM 25914 und DSMZ 25915, oder Zelllysate, Derivateoder Mutanten davon, haben insbesondere die Fähigkeit zur Koaggregation, bevorzugt spezifischen Bindung von zumindest einem pathogenen Mikroorganismen, welcher ausgewählt ist aus der Gruppe *Staphylococcus aureus* und/oder *Pseudomonas aeruginosa.* Es war völlig überraschend, dass die bevorzugten Milchsäurebakterien keine Koaggregation oder Bindung der kommensalen Hautbakterien oder Mikroorganismen, wie *Corynebacterium jeikeium, Mikrokokkus luteus, Propionibakterium acnes* oder vor allem *Staphylococcus epidermidis* bewirken. Beispielsweise *Staphylococcus epidermidis* stellt ein weitestgehend unauffälliger Kommensale der Hautflora dar. Im Zusammenspiel der unterschiedlichen, in oder auf der Haut vorhandenen Mikroorganismen ist er in der gesunden Hautflora vieler Säugetiere vorhanden, und steht - zumindest bei gesunder Haut - mit diesen im mikrobiellen Gleichgewicht. Daher ist eine Beeinflussung dieses Bakteriums, bspw. durch eine Aggregation und dadurch Beeinflussung mit anderen Mikroorganismen, die gezielt in der Körperhygiene eingesetzt werden sollen, im Rahmen der vorliegenden Erfindung nicht bevorzugt.

Das heißt, die bevorzugten Milchsäurebakterien koaggregieren spezifisch mit den pathogenen Bakterien *Staphylococcus aureus* und/oder *Pseudomonas aeruginosa.* Nichts dergleichen geht aus dem Stand der Technik hervor. Im Stand der Technik sind lediglich Milchsäurebakterien beschrieben, die auch eine Bindung an kommensale Mikroorganismen zeigen, so dass diese nicht ohne Nebenwirkungen hervorzurufen, eingesetzt werden können. Im Gegensatz dazu zeigen die bevorzugten Milchsäurebakterien keine Bindung oder Koaggregation von kommensalen Hautbakterien oder sonstigen apathogenen Mikroorganismen, die die Hautflora besiedeln. Gemäß einer weiteren bevorzugten Ausführungsform besitzen die erfindungsgemäßen Milchsäurebakterien nicht die Fähigkeit, an kommensale Hautkeime,

Dem Fachmann ist bekannt, dass die gesunde Haut mit Mikroorganismen, wie Bakterien und Pilzen, die als Kommensalen bzw. Mutualen dicht besiedelt. Die Mikroorganismen stellen einen natürlichen Bestandteil der Hautoberfläche dar und werden insbesondere als Hautflora zusammengefasst. Die Mikroorganismen, die unter dem Begriff der Hautflora zu subsumieren sind, stellen eine wichtige Voraussetzung dar, um die Haut selbst und den Organismus als Ganzen vor pathogenen Keimen zu schützen und sind Teil des Mikrobioms. Diesbezüglich ist es besonders vorteilhaft, dass die bevorzugten Milchsäurebakterien die Hautflora nicht aus dem Gleichgewicht bringen, sondern lediglich an die pathogenen Bakterien *Staphylococcus aureus* und/oder *Pseudomonas aeruginosa* binden, bzw. diese koaggregieren.

Die Mikroorganismen zeigen insbesondere spezifische adhäsive Eigenschaften und bilden Koaggregate mit den pathogenen Keimen, *Staphylococcus aureus* und/oder *Pseudomonas aeruginosa.* Es ist jedoch auch bevorzugt, dass die Milchsäurebakterien auch andere pathogene Spezies der Mikroorganismen *Staphylococcus* oder *Pseudomonas* spezifisch koaggregieren oder mit diesen zumindest interagieren.

Nicht zuletzt zeigen die erfindungsgemäßen Mikroorganismen biofilmverhindernde Eigenschaften. Dadurch, dass die bevorzugten Milchsäurebakterien die pathogenen Bakterien koaggregieren, bzw. adhäsive Eigenschaften gegenüber diesen zeigen, können die pathogenen Keime maskiert werden, was zur Verdeckung vieler Pathogenitätsfaktoren und somit zur Reduktion der bakteriellen Belastung und/oder zur Inhibierung der Biofilmbildung führt, z.B. durch Maskierung bzw. Abbindung der entsprechenden Oberflächenadhäsine der pathogenen Bakterien.

Auch wenn die Erfindung insbesondere eine Gruppe Milchsäurebakterien betrifft, ist die Einheitlichkeit der anmeldungsgemäßen Lehre gegeben. Die beanspruchten Mikroorganismen haben eine gemeinsame Eigenschaft oder Wirkung. Die Summe der strukturellen oder funktionellen Gemeinsamkeiten führt zu dem funktionellen Zusammenhang der Koaggregation von *Staphylococcus aureus* und/oder *Pseudomonas aeruginosa,* der Nicht-Bindung kommensaler Mikroorganismen der Haut und der Verhinderung von Biofilmbildung bzw. Zerstörung etablierter Biofilme durch *Staphylococcus aureus* und/oder *Pseudomonas aeruginosa.* Die gemeinsamen Merkmale stellen daher keine willkürliche Summe von Merkmalen dar, sondern sind sozusagen der gemeinsame Fingerprint der beanspruchten Mikroorganismen, der vorteilhafterweise die Tauglichkeit der Mikroorganismen für den Zweck ermöglicht und charakterisiert.

Die bevorzugten Milchsäurebakterien ausgewählt aus der Gruppe umfassend die Stämme DSM 25906, DSM 25907, DSM 25908, DSM 25909, DSM 25910, DSM 25911, DSM 25912, DSM 25913, DSM 25914 und DSMZ 25915 oder Derivaten, Zelllysate oder Mutanten davon sind durch den funktionellen Zusammenhang dergestalt miteinander zu einer einheitlichen erfinderischen Idee verbunden, dass sie Eigenschaften bzw. Wirkungen gemein haben, nämlich dass sie spezifisch die pathogenen Bakterien *Staphylococcus aureus* und/oder *Pseudomonas aeruginosa* koaggregieren, keine kommensale Mikroorganismen der Haut binden und zudem die Biofilmbildung durch *Staphylococcus aureus* und/oder *Pseudomonas aeruginosa* verhindern, bzw. die Biofilme zerstören. Es war völlig überraschend, dass eine Gruppe Milchsäurebakterien identifiziert werden konnten, die identische vorteilhafte Eigenschaften aufweisen. Im Stand der Technik sind keine Bakterien, insbesondere Milchsäurebakterien beschrieben, die alle Eigenschaften vereinen und zudem apathogen sind und zu keiner Schädigung oder Beeinflussung der natürlichen Hautflora führen. Es hat sich zudem gezeigt, dass eine Applikation der erfindungsgemäßen Milchsäurebakterien, sei es als Zusammensetzung oder anderweitig, die Regeneration einer durch *Staphylococcus aureus* und/oder *Pseudomonas aeruginosa* geschädigten Hautflora beschleunigt. Die Ursache hierfür ist bis jetzt unbekannt, soll aber durch weitere Versuche identifiziert werden.

Ein weiterer überraschender Vorteil der erfindungsgemäßen Milchsäurebakterien ist, dass diese auch prophylaktisch verwendbar sind. Das heißt, die mit Milchsäurebakterien, bzw. eine diese umfassende Zusammensetzung können auf eine gefährdete Hautregionen bzw. bei gefährdeten Personen- oder Tiergruppen prophylaktisch aufgebracht werden, ohne dass hierdurch eine Schädigung der Haut oder der Hautflora erfolgt. Es ist z. B. bekannt, dass sich bei Personen, die für einen längeren Zeitraum liegend gelagert werden müssen, offene Wunden an Druckstellen bilden. Erste Versuche haben gezeigt, dass eine prophylaktische Behandlung dieser Regionen eine Entstehung von offenen Wunden vermeidet oder diese zumindest vor weiteren Infekten durch *Staphylococcus aureus* und/oder *Pseudomonas aeruginosa* schützt.

Im Sinne der Erfindung wird die Haut in bevorzugten Ausführungsformen insbesondere als äußeres Organ des menschlichen oder tierischen Organismus verstanden, welches der Abgrenzung von innen und außen dient. Zu einem Hautareal im Sinne der Erfindung gehören in bevorzugten Ausführungsformen Bestandteile der Oberhaut, der Lederhaut oder der Unterhaut. Die Oberhaut (Epidermis) besteht erfindungsgemäß aus folgenden Schichten: Hornschicht (Stratum corneum), Leuchtschicht (Stratum lucidum), Körnerschicht (Stratum granulosum), Stachelzellschicht (Stratum spinosum) und/oder Basalschicht (Stratum basale). Jede Modifikation von Zellen in diesem Bereich ist eine Zellmodifikation in einem Hautareal im Sinne der Erfindung. Die Lederhaut (Dermis, Corium), die ebenfalls ein Bestandteil der erfindungsgemäßen Hautareale sein kann, besteht vorwiegend aus Bindegewebsfasern und dient der Ernährung und Verankerung der Epidermis. Das kapillarisierte Blutgefäßsystem in der Grenzzone zur Epidermis gehört mit zum Hautareal im Sinne der Erfindung ebenso wie die Talg- und Schweißdrüsen. Die Dermis im Sinne der Erfindung kann in ein Stratum papillare und in ein Stratum reticulare unterteilt werden. Weiterhin kann ein Hautareal im Sinne der Erfindung jedes Areal, d. h. auch jeder Ort in oder an der Unterhaut (Subcutis) oder eines Gewebes im Inneren des Körpers oder einer jeden Organes oder Organbestandteiles sein. Eine Gewebebarriere, die ein Organ von den umgebenden Strukturen abgrenzt, kann eine Haut im Sinne der Erfindung sein. Weiterhin werden durch den erfindungsgemäßen Begriff des Hautareals auch Hautanhanggebilde umfasst, wie Haare, Talgdrüsen, Haarbalgmuskeln, Nägeln, Hörner und Schweißdrüsen, insbesondere die ekkrinen und die apokrinen Schweißdrüsen, aber auch die Milchdrüsen. Jede Zellmodifikation, insbesondere ein vom normalen abweichendes Zellwachstum, kann mit den erfindungsgemäßen Mitteln behandelt werden, bevorzugt ohne auf die äußeren Hautareale beschränkt zu sein. Die Hautareale im Sinne der Erfindung können aber auch die Leistenhaut betreffen, wie sie an den Fingern oder an der Fußsohle auftreten oder aber die Felderhaut und die hiermit assoziierten Hautanhangsgebilde.

Die Verträglichkeit der Milchsäurebakterien der Haut ist eine Voraussetzung für eine erfolgreiche Behandlung von Hautinfektionen und bakteriell verursachten Entzündungen in Wunden oder sonstigen Erkrankungen oder Beschwerden, bei denen Mikroorganismen aus der Gruppe der *Staphylococcen* oder *Pseudomonaden* vorkommen.

Die bevorzugte Zusammensetzung kann insbesondere in einer Seife, einer Lotion, einem Puder, einem Syndet, einem Schaum, einem Stift, einer Emulsion, einem Spray, einem Creme, einem Gel, einem Shampoo, einer Flüssigseife oder einem Deo enthalten sein. Ferner ist es bevorzugt, die Zusammensetzung, insbesondere als Probiotikum zu verwenden, das als Wasch-, Spül-, Reinigungs-, oder Desinfektionsmitteln (z.B. Seifen, Pulvern, Pasten, Lösungen, Emulsionen, Lotionen), Reinigungs- und/oder Desinfektionstüchern, Shampoos, Spülungen oder Anwendungen für Haut, Haare und/oder Kopfhaut, Cremes, Salben, Hautreinigungs- und/oder -pflegelotionen, in Lösungen (z.B. als Tropfen, Spray, Spülung) zur Anwendung in oder an Augen, Ohren, Mund, Nasen-, Rachenraum zugesetzt und/oder in Bandagen oder Wundauflagen eingebracht werden kann, um die Bildung von pathogenen Leitkeimen zu unterbinden, diese zu binden, diese als Aggregat zu entfernen und/oder sie zu hemmen bzw. abzutöten und sie so zu reduzieren.

Es war völlig überraschend, dass die Vorteile der erfindungsgemäßen Zusammensetzung noch einmal verbessert werden können, indem es in die genannten galenischen Formen eingebracht wird. Dem Fachmann sind weitere Formulierungskonzepte für das Einbringen der erfindungsgemäßen Zusammensetzung z. B. in Trägersubstanzen bekannt wie z. B. Emulsionen, oder andere Produkte zur dermalen Applikation wie z. B. flüssige Formen, die bevorzugt wasserfrei oder wasserhaltig sein können, wobei die wasserhaltigen erfindungsgemäß in Einphasensysteme und in Mehrphasensysteme unterschieden werden können. Weiterhin können halbfeste Formen, die wasserfrei oder wasserhaltig sind, eingesetzt werden, wobei wiederum eine Einteilung in Einphasensysteme und Mehrphasensysteme bei den wasserhaltigen halbfesten Formen möglich ist. Weiterhin können bevorzugt feste Formen eingesetzt werden, die lipophil oder hydrophil sind. Beispiele für solche Formen sind neben den genannten z. B. Fettsalben, Schäume, Puder, Stifte, Gelcremen, Hydrodispersionsgele, dünnflüssige Emulsionen, Lotionen, Salben, Sprays und Cremes. Hierbei ist dem Fachmann bekannt, dass sich derartige Trägersubstanzen zum einen vom Hautgefühl her in reichhaltig/wertvolle und frische und leichte unterscheiden lassen und zum anderen im Hinblick auf die Viskosität in niedrigviskose und in hochviskose. Nanoemulsionen oder Öle bzw. Oleogele weisen eher eine niedrige Viskosität auf, wohingegen Hydrogele oder Hydrocremen bzw. O/W-Emulsionen oder W/O-Emulsionen eine hohe Viskosität aufweisen. Wenn flüssige Applikationsformen zum Einsatz kommen, können diese - wie oben ausgeführt - in wasserfreie und wasserhaltige Systeme unterteilt werden. Bei den wasserfreien Systemen sind insbesondere apolare Systeme, polare Systeme ohne Emulgatoren und polare Systeme mit Emulgatoren bevorzugt. Bei den wasserhaltigen Systeme sind einphasige Systeme wie Lösungen und Mikroemulsionen bevorzugt, bei den mehrphasigen Systemen sind multiple Emulsionen, W/O-Emulsionen oder O/W-Emulsionen bevorzugt. Bei den Fest/Flüssig-Systemen sind bevorzugte Formen die Suspensionen oder die Flüssig/Fest/Flüssig-Systeme wie Suspensions-/Emulsionssysteme. Dem Fachmann sind verschiedene Möglichkeiten bekannt, derartige Träger bereitzustellen. Bei den O/W-Emulsionen sind bevorzugte galenische Leitsubstanzen O/W-Emulgatoren, W/O-Emulgatoren, flüssige hydrophile Bestandteile und flüssige lipophile Bestandteile. Bei den W/O-Emulsionen sind bevorzugte galenische Leitsubstanzen W/O-Emulgatoren, O/W-Emulgatoren, flüssige und halbfeste lipophile Bestandteile, Gelbildner, flüssige hydrophile Bestandteile und/oder Salze.

Bei den halbfesten bevorzugten Trägersubstanzen sind wasserfreie Systeme wie wasserhaltige Systeme für unterschiedliche Anwendungen bevorzugt. Wasserfreie Systeme können aus apolaren Systemen bestehen oder aus polaren Systemen ohne Emulgatoren wie Lipogele, Oleogele oder Polyethylenglycolgele bzw. aus apolaren Systemen mit Emulgatoren auf O/W-Absorptionsgrundlagen oder W/O-Absorptionsgrundlagen. Die wasserhaltigen Systeme können bevorzugt aus einphasigen Systemen wie Hydrogelen oder Mikroemulsionsgelen oder mehrphasigen Systemen wie O/W-Cremes, W/O-Cremes oder amphiphilen Systemen bestehen. Die bevorzugten halbfesten Zubereitungen sind im Temperaturbereich zwischen Raumtemperatur und Hauttemperatur streichfähige Zubereitungen zur Anwendung auf der Haut oder auf Schleimhäuten, die eine lokale Wirkung ausüben, Wirkstoffe transportieren oder eine erweichende oder schützende Wirkung auf die Haut ausüben. Bevorzugte Zubereitungen sind Salben im engeren Sinne, Cremes, Gele und/oder Pasten. Neben den Salben, Cremes, Gelen und Pasten können auch Oleogele als halbfeste, transparente Einphasensysteme eingesetzt werden. Dem Fachmann sind beispielsweise aus dem US-Patent 6,187,323 oder Aiache et al. 2001 verschiedene wasserfreie Compounds zur Formulierung halbfester Systeme bekannt, wie beispielsweise die Verbindung aus einem Oleogel und eine Hydrogel, welche erfindungsgemäß als Bi-Gel bezeichnet werden kann. Weiterhin können Hydrodispersionsgele oder verschiedene Lipide herangezogen werden, um erfindungsgemäße Trägersubstanzen bereitzustellen. Beim Einsatz von Lipiden können silizi-umorganische Verbindungen ebenso wie kohlenstofforganische Verbindungen zur Bereitstellung von Lipidphasen in dispersen Systemen zum Einsatz kommen, wobei kohlenstofforganische Verbindungen beispielsweise mithilfe von nicht-hydrolisierbaren Lipiden oder hydrolisierbaren Lipiden (Glyzerine), Wachsester bereitgestellt werden können. Vorteile derartiger Systeme sind die verbesserte Geschmeidigkeit der Haut und die Steigerung ihrer Elastizität sowie ihre Fähigkeit, je nach Zusammensetzung der Lipide freisetzungs- und penetrationsbeeinflussend wirken zu können.

Dem Fachmann ist bekannt, welche Lipide er einsetzen muss, um beispielsweise die Penetration innerhalb eines Zeitparameters zu steigern oder zu vermindern.

Weitere bevorzugte Trägersubstanzen sind beispielsweise Hydrodispersionsgele bzw. Mikrokapseln, Mikrosphärulen oder Pellets (Macrobeads). Die genannten Träger dienen der Stabilitätserhöhung und stellen eine Mindestanwendungsdauer auf der Haut sicher. Die bevorzugten halbfesten Einphasensysteme können mithilfe der folgenden galenischen Leitsubstanzen bereitgestellt werden: flüssige hydrophile Bestandteile, insbesondere Wasser und (Poly-)Alkohole, hydrophile Gelbildner, Salzbildner sowie W/O-Emulgatoren, O/W-Emulgatoren, flüssige, halbfeste und feste lipophile Bestandteile sowie lipophile Gel- bzw. Gerüstbildner. Dem Fachmann ist bekannt, wie er diese Substanzen kombinieren muss, um eine bestimmte Wirkung zu erzielen.

Dem Fachmann sind weitere galenische Zubereitungen für dermale Produkte bekannt. Anmeldungsgemäß können beispielsweise alle in Daniels and Knie in der JDDG; 2007, 5: 367-383 offenbarten galenischen Verbindungen eingesetzt werden. Dem Fachmann ist bekannt, dass unterschiedliche galenische Zubereitungen unterschiedlich auf der Haut wirken und die erfindungsgemäße Zusammensetzung unterschiedlich stark in die Haut eintragen. Der Inhalt der JDDG; 2007, 5: 367-383 ist in den Offenbarungsgehalt der anmeldungsgemäßen Lehre mit aufgenommen. Bevorzugte erfindungsgemäße Produkte sind beispielsweise lipophile oder hydrophile Lösungen, lipophile oder hydrophile Emulsionen, lipophile oder hydrophile Suspensionen, spezielle flüssige Zubereitungen, hydrophobe oder hydrophile Salben, Wasseremulgierende Salben, lipophile, hydrophile oder amphiphile Cremen, Hydrogele, hydrophobe oder hydrophile Pasten und/oder Puder.

Versuche haben gezeigt, dass die bevorzugten Milchsäurebakterien, insbesondere Lactobacillus Zellen beim Kontakt mit *Staphylococcus aureus* bzw. *Pseudomonas aeruginosa* Zellen Koaggregate bilden. Durch die Bildung von Co-Aggregaten wird *Staphylococcus aureus* bzw. *Pseudomonas aeruginosa* daran gehindert, insbesondere in die Hautwunden einzudringen oder sich auf der Haut anzusiedeln und zu etablieren, zu adhäsieren und Biofilme zu bilden. Die *Staphylococcus aureus* bzw. *Pseudomonas aeruginosa* Zellen, insbesondere deren Zelloberfläche, werden durch die Milchsäurebakterien, insbesondere Lactobacillus Zellen maskiert, so dass die *Staphylococcus aureus* bzw. *Pseudomonas aeruginosa* Zellen bevorzugt nicht mehr in der Lage sind, an die Hautepithelzellen zu binden. Durch die verhinderte Bindung von *Staphylococcus aureus* bzw. *Pseudomonas aeruginosa* an die Hautepithelzellen bleiben Entzündungsreaktionen aus bzw. Hautirritationen werden reduziert oder umgangen. Die *Staphylococcus aureus* bzw. *Pseudomonas aeruginosa* Zellen, insbesondere deren Zelloberfläche, werden durch die Milchsäurebakterien, insbesondere Lactobacillus Zellen gebunden. Dabei entstehen Zell-Koaggregate, bestehend aus Lactobacillus Zellen und/oder *Staphylococcus aureus* und/oder *Pseudomonas aeruginosa* Zellen, welche einfacher und effizienter und effektiver von Haut, Wunden und Oberflächen im allgemeinen (Fell, Federn, Stahl-, Kunststoff-, Metalloberflächen) entfernt (abgewaschen) werden können, im Vergleich zu einzelnen oder in kleinen Aggregaten vorliegenden *Staphylococcus aureus* bzw. *Pseudomonas aeruginosa* Zellen.

Im Sinne der Erfindung umfassen probiotische Mikroorganismen Zellen, welche vorteilhafte Wirkungen auf menschliche und/oder tierische Körper zeigen. Eine bevorzugte Zusammensetzung wird als probiotische Zusammensetzung verwendet und enthält, Milchsäurebakterien, die eine vorteilhafte Wirkung auf menschliche und/oder tierische Körper zeigen. Vorteilhafte Wirkungen können insbesondere in der Verbesserung der Hautflora bestehen. Insbesondere können in der Hautflora unerwünschte Mikroorganismen, wie *Staphylococcus aureus* und/oder *Pseudomonas aeruginosa* durch unmittelbare Wechselwirkungen mit den probiotischen Mikroorganismen und den unerwünschten Mikroorganismen und insbesondere durch mittelbare Wechselwirkungen auf Grund von Hemmungen des Metabolismus des unerwünschten Mikroorganismus durch Expressionsprodukte des probiotischen Mikroorganismus gehemmt werden. Es konnte durch Versuche gezeigt werden, dass die pathogenen Keime *Staphylococcus aureus* und/oder *Pseudomonas aeruginosa* nach einer Koaggregation durch die bevorzugten Milchsäurebakterien kein Wachstum, d. h. keine Vermehrung der Zellmasse mehr aufweisen, sondern die Zellen insbesondere maskiert, in Koaggregaten gebunden und/oder abgetötet werden.

Die, Mutanten oder Derivate der beschriebenen Milchsäurebakterien, welche insbesondere durch biologische, chemische oder physikalische Behandlung der Milchsäurebakterien hergestellt werden, zeigen überraschenderweise auch nach der Behandlung die vorteilhaften Eigenschaften.

Außerdem war es überraschend, dass die Milchsäurebakterien, , Derivate, Mutanten, oder Kombinationen hiervon auch nach physikalischer, chemischer und/oder biologischer Inaktivierung die vorteilhaften Eigenschaften besitzen. Beispielsweise konnten die bevorzugten Stämme, nämlich DSM 25906, DSM 25907, DSM 25908, DSMZ 25909, DSM 25910, DSM 25911, DSM 25912, DSM 25913, DSM 25914 und DSMZ 25915 auch nach Hitzebehandlung bei 70° Celsius für 20 Minuten oder einer Behandlung mit Ultraschall eine Koaggregation der pathogenen Keime bewirken, die Biofilmbildung verhindern und zeigen zudem keine Bindung kommensaler Mikroorganismen. Somit können die Milchsäurebakterien, , Derivate, Mutanten oder Kombinationen hiervon vorteilhafterweise auch inaktiv, insbesondere nicht-lebend in einer bevorzugten Ausführung der Zusammensetzung vorliegen. Hierdurch kann die Haltbarkeit und Nutzbarkeit der Zusammensetzung erheblich verlängert werden. Außerdem kann die Zusammensetzung in weiteren Anwendungsbereichen Verwendung finden, die die Nutzung von lebenden Mikroorganismen nicht zulassen. Es war völlig überraschend, dass die Milchsäurebakterien in der Zusammensetzung inaktiviert, lebend oder nicht-lebend vorliegen und nichtsdestotrotz die pathogenen Mikroorganismen spezifisch binden, bzw. diese koaggregieren. Die Zusammensetzung kann vorteilhafterweise als Nahrungsmittel, Nahrungsmittelzusatz, Pharmazeutikum, Medizinprodukt, Kosmetikum, Reinigungsmittelzusatz und als Tiernahrung oder Getränk eingesetzt werden.

Es ist eine Zusammensetzung bevorzugt, die die erfindungsgemäßen Milchsäurebakterien oder Zelllysate, Mutanten oder Derivate davon enthält, welche die Fähigkeit zur Koaggregation mindestens eines pathogenen Mikroorganismus aufweisen, der ausgewählt ist aus der Gruppe *Staphylococcus aureus und/oder Pseudomonas aeruginosa,* wobei die Zusammensetzung für die Körperhygiene, -therapie und/oder -prophylaxe verwendet wird.

Die bevorzugten Mikroorganismen sind Vertreter der Gattung oder Ordnung der Milchsäurebakterien, mithin also Gram-positive Bakterien, die Milchsäure durch Fermentation von Glucose erzeugen. Die erfindungsgemäßen Mikroorganismen zeichnen sich einerseits dadurch aus, dass sie die Fähigkeit zur spezifischen Koaggregation von zumindest einem pathogenen Mikroorganismus aufweisen, der ausgewählt ist aus der Gruppe *Staphylococcus aureus* oder *Pseudomonas aeruginosa.* Diese Bindung führt zu einer Bildung von Aggregaten aus den erfindungsgemäßen Mikroorganismen und den spezifisch gebundenen, pathogenen Mikroorganismen. Durch die Koaggregat-Bildung können letztere, also die pathogenen Mikroorganismen mechanisch, bspw. durch Abspülen, gezielt und leicht entfernt werden, was durch bisher bekannte Maßnahmen nicht möglich war. Zell-Koaggregate, bestehend aus Lactobacillus Zellen und/oder *Staphylococcus aureus* und/oder *Pseudomonas aeruginosa* Zellen können einfacher und effizienter und effektiver von Haut, Wunden und Oberflächen im allgemeinen (Fell, Federn, Stahl-, Kunststoff-, Metalloberflächen) entfernt (abgewaschen) werden können, im Vergleich zu einzelnen oder in kleinen Aggregaten vorliegenden *Staphylococcus aureus* bzw. *Pseudomonas aeruginosa* Zellen.

Unter dem Ausdruck "spezifische Bindung" oder "Koaggregation" wird im Sinne der Erfindung sowie üblicherweise im Gebiet der Mikrobiologie und Hygiene, insbesondere der humanen Mikrobiologie und Körperhygiene die gegenseitige Erkennung und Adhäsion von Zellen, die genetisch unterschiedlichen Typen von Zellen angehören, verstanden. Hierzu exprimieren Bakterien auf ihrer Zelloberfläche Rezeptoren und Strukturen für Adhäsine auf anderen Zelltypen, die für die Anheftung zwischen den Zellen verwendet werden. Diese Adhärenz spielt für die Besiedelung mit pathogenen wie auch mit kommensalen Mikroorganismen eine herausragende Rolle, so dass ein Eingriff in die Adhärenz zu weitreichenden Konsequenzen führen kann. Dadurch, dass die erfindungsgemäßen Mikroorganismen die Fähigkeit zur spezifischen Bindung, insbesondere Koaggregation mit zumindest einem Mikroorganismus der Gruppe *Staphylo-*

*coccus aureus* oder *Pseudomonas aeruginosa* aufweisen, können sich Aggregate aus den erfindungsgemäßen Mikroorganismen und den pathogenen Mikroorganismen bilden. Die entstehenden Koaggregate können leicht, bspw. durch Spülen, von Oberflächen, der Haut, Geweben und/oder einem sonstigen Besiedlungsort oder - reservoir entfernt werden, so dass die Zahl der pathogenen Mikroorganismen deutlich reduziert wird. Des Weiteren wird durch Maskierung der Oberflächenstrukturen der pathogenen Mikroorganismen eine initiale bzw. erneute Anheftung an Oberflächen, der Haut, Geweben und/oder sonstigen Besiedlungsorten oder -reservoirs verhindert bzw. reduziert. Wenn Zellen untereinander binden und Agglomerate bilden, wird dieser Vorgang insbesondere als Aggregation. Wenn bei dieser Aggregat-Bildung nur eine Zellart beteiligt ist, wird das als Autoaggregation oder Selbstaggregation bezeichnet. Sind bei der Aggregat-Bildung mindestens zwei verschiedene Zellarten beteiligt, bezeichnet man den Vorgang insbesondere als Koaggregation.

Im Sinne der Erfindung wird mit "spezifische Bindung von zumindest einem pathogenen Mikroorganismus" insbesondere die Eigenschaft der erfindungsgemäßen Mikroorganismen verstanden, mindestens ein Bakterium aus der Gruppe umfassend *Staphylococcus aureus* und/oder *Pseudomonas aeruginosa* spezifisch zu binden.

Gemäß einer bevorzugten Ausführungsform der erfindungsgemäßen Mikroorganismen zeichnen sich diese ferner dadurch aus, dass die Fähigkeit zur spezifischen Bindung an zumindest einen pathogenen Mikroorganismus auch nach einer biologischen, chemischen oder physikalischen Behandlung, z. B. einer Hitzebehandlung bei mind. 70 °C besteht. Das heißt, die bevorzugten Milchsäurebakterien können bevorzugt inaktiv in einer bevorzugten Zusammensetzung vorliegen, da die Fähigkeit zur spezifischen Wechselwirkung oder Bindung mit pathogenen Bakterien, insbesondere *Staphylococcus aureus* und/oder *Pseudomonas aeruginosa* nicht beeinflusst ist und somit eine Bindung oder Wechselwirkung hergestellt werden kann.

Inaktivierte oder nicht-lebende Milchsäurebakterien Zellen können besonders vorteilhaft sein, da keine metabolische Aktivität von den Milchsäurebakterien Zellen ausgehen kann.

Gemäß einer weiteren bevorzugten Eigenschaft der Milchsäurebakterien können sich die erfindungsgemäßen Mikroorganismen daher auch durch eine Hitzestabilität auszeichnen, und eine Behandlung mit hohen Temperaturen, vorzugsweise von mindestens etwa 60 °C, bevorzugter von mindestens 65 °C und noch bevorzugter von mindestens 70 °C, über einen Zeitraum von mindestens 20 Minuten, vorzugsweise von 25 Minuten und noch bevorzugter von mindestens ca. 30 Minuten, überstehen und bezüglich ihrer Fähigkeit zur Koaggregation von zumindest einem der genannten pathogenen Mikroorganismen unverändert bleiben.

In einer bevorzugten Ausführungsform der Zusammensetzung sind Milchsäurebakterien bevorzugt, die inaktiviert, lebend oder nicht-lebend sind oder Teile und Fragmente, z.B. enzymatische oder mechanische Spaltprodukte (z.B. French Press etc.) oder Stoffwechselprodukte dieser, soweit diese noch die Fähigkeit zur Koaggregation und/oder Biofilmverhinderung aufweisen. Es ist zudem bevorzugt, dass die Milchsäurebakterien in verkapselter, sprühgetrockneter und/oder lyophilisierter Form eingesetzt werden, d. h. in verkapselter, sprühgetrockneter und/oder lyophilisierter Form in einer bevorzugten Zusammensetzung vorliegen. Außerdem kann es vorteilhaft sein, wenn die Milchsäurebakterien in Form eines Zellaufschlusses eingesetzt werden.

Ferner ist bevorzugt, wenn die Fähigkeit der erfindungsgemäßen Milchsäurebakterien zur spezifischen Bindung an die pathogenen Mikroorganismen auch bei einem pH-Wert von zwischen ca. 3 bis 8 besteht. Das heißt, die Milchsäurebakterien können sich in einem Milieu befinden, welches einen pH-Wert von 3 bis 8 aufweist und noch ihre Fähigkeit zur Bindung von *Staphylococcus aureus* und/oder *Pseudomonas aeruginosa* aufweisen. Dem Fachmann ist bekannt, dass die Haut einen leicht sauren pH-Wert aufweist. Diesbezüglich ist es besonders vorteilhaft, dass die bevorzugten Milchsäurebakterien ihre Fähigkeit zur Koaggregation von *Staphylococcus aureus* und/oder *Pseudomonas aeruginosa* auch in einem breiten pH-Wert-Bereich aufweisen. Der pH-Wert der Haut kann sich z. B. durch Kosmetika oder auch im Bezug auf verschiedene Körperregionen unterscheiden. Die bevorzugten Milchsäurebakterien können vorteilhafterweise in einem großen pH-Wert-Bereich eingesetzt werden, bzw. zeigen in dem bevorzugten Bereich die bevorzugten Eigenschaften. Die bevorzugten Milchsäurebakterien können somit universell in unterschiedlichen Körperbereichen eingesetzt werden. Ferner stellt die beschriebene Erfindung eine Zusammensetzung dar, insbesondere ein innovatives Probiotikum, welche auch nach Proteasebehandlung, z.B. Trypsin, Trypsin TPCKbehandelt, Lysozym, Proteinase K, Pronase, Thrombin, PNGase, Pepsin, Chymotrypsin, Papain) Koaggregatonseigenschaften aufweist.

Überraschenderweise zeigen die erfindungsgemäßen Milchsäurebakterien in einem breiten Temperaturbereich von ca. 25°C - 42° C Koaggregatonseigenschaften insbesondere mit *Staphylococcus aureus* und/oder *Pseudomonas aeruginosa.*

Dem Fachmann wird hierbei, sowie bei sämtlichen in der vorliegenden Erfindung aufgeführten Bereichsangaben, die durch die Begriffe "ca." oder "etwa" gekennzeichnet sind, klar sein, dass mit dem Ausdruck "ca." oder "etwa" nicht die exakte Zahlenangabe gemeint sein muss, sondern dass auch geringfügige Abweichungen nach oben oder unten bezüglich der angegebenen Zahl noch in den Rahmen der vorliegenden Erfindung liegen.

Die Bindung der erfindungsgemäßen Milchsäurebakterien an die aufgeführten pathogenen Mikroorganismen führt bevorzugt zu einer Hemmung des Wachstums dieser pathogenen Mikroorganismen.

Es war überraschend, dass durch die Bindung der pathogenen Mikroorganismen und den erfindungsgemäßen Milchsäurebakterien ein Aggregat gebildet wird, das insbesondere nach bevorzugt 5 bis 100 Minuten bei Raumtemperatur ohne Agitation als Sediment vorliegt. Aerobe, hautpathogene Bakterien in Wunden sind insbesondere *Staphylococcus aureus* und *Pseudomonas aeruginosa.* Normalerweise dient die Entzündungsphase der Wundheilung zur Bekämpfung potentiell pathogener Mikroorganismen und zur Zellregeneration. Allerdings treten nicht heilende Wunden oft bei bereits immunsupprimierten Patienten auf, die eine verminderte Entzündungsantwort aufweisen. Diese geschwächte Immunantwort kann die primären Wundbakterien nicht mehr effektiv abwehren und so dringen die Bakterien in die Wunde ein und bilden Gemeinschaften, die als Biofilme organisiert sind. Diese Biofilme sind nicht nur resistent gegen das Abwehrsystem des Wirtes sondern auch planktonische Zellen oder Mikrokolonien.

In einer bevorzugten Ausführungsform weisen die bevorzugten Milchsäurebakterien Mutanten, Derivat oder Kombinationen davon bevorzugt zumindest eines der folgenden Merkmale auf: a) Hitzestabilität oder stabil nach biologischer, chemischer und/oder physikalischer Behandlung oder b) Fähigkeit zur Hemmung der Biofilmbildung durch *Staphylococcus aureus* oder *Pseudomonas aeruginosa.* Es war völlig überraschend, dass die erfindungsgemäßen Milchsäurebakterien auch nach biologischer, chemischer und/oder physikalischer Behandlung ihre Fähigkeiten erhalten, *Staphylococcus aureus* und/oder *Pseudomonas aeruginosa* spezifisch zu binden.

Eine weitere Eigenschaft der erfindungsgemäßen Milchsäurebakterien ist die Fähigkeit zur Hemmung der Biofilmbildung durch *Staphylococcus aureus* und/oder *Pseudomonas aeruginosa.* Diese Bakterien bilden extrem zähe Biofilme, die gegenüber Ultraschall, Detergentien, Proteasen oder Hitze resistent sind, und sich auch schon als resistent gegenüber antimikrobiellen Substanzen erwiesen haben. Vorteilhafterweise besitzt der erfindungsgemäße Mikroorganismus die Eigenschaft, die Biofilm-Bildung durch *Staphylococcus aureus* und/oder *Pseudomonas aeruginosa* zu hemmen; aufgrund der Hemmung der Biofilm-Bildung können diese pathogenen Bakterien biologische, wie auch anorganische Oberflächen nicht mehr besiedeln, und können konsequenterweise auch keine Erkrankungen auslösen.

Daher wird im Sinne der Erfindung insbesondere unter "Hemmung der Biofilm-Bildung durch *Staphylococcus aureus* und *Pseudomonas aeruginosa*" jede Eigenschaft der erfindungsgemäßen Milchsäurebakterien verstanden, derart mit *Staphylococcus aureus* und *Pseudomonas aeruginosa* zu interagieren, d.h. an diese zu binden oder sie anderweitig zu beeinflussen, so dass diese keinen Biofilm mehr bilden können.

Gemäß einer bevorzugten Ausführungsform kann der erfindungsgemäße Mikroorganismus daher mindestens eine der aufgeführten Eigenschaften Resistenz gegenüber biologischer, chemischer und/oder physikalischer Behandlung, Fähigkeit zur spezifischen Koaggregation zumindest einen pathogenen Mikroorganismus und/oder Fähigkeit zur Hemmung der Biofilm-Bildung durch *Staphylococcus aureus* oder *Pseudomonas aeruginosa* aufweisen. Bevorzugte Kombination der Eigenschaften sind z. B. Resistenz gegenüber biologischer, chemischer und/oder physikalischer Behandlung und Fähigkeit zur Bindung an zumindest e *Staphylococcus aureus und/oder Pseudomonas aeruginosa*, oder Resistenz gegenüber biologischer, chemischer und/oder physikalischer Behandlung und Hemmung der Biofilm-Bildung durch *Staphylococcus aureus* oder *Pseudomonas aeruginosa.*

Mit dem Ausdruck "Mikroorganismus, der zu der Gattung oder Ordnung der Milchsäurebakterien gehört" werden vorliegend, wie bereits aufgeführt, auch Derivate oder Mutanten davon umfasst, welche die hierin beschriebenen Charakteristika bzw. Merkmale oder Eigenschaften der erfindungsgemäßen Mikroorganismen noch besitzen. Vorzugsweise handelt es sich bei den erfindungsgemäßen Milchsäurebakterien um Bakterien der Art *Lactobacillus gasseri*, *Lactobacillus crispatus* und *Lactobacillus ingluviei.*

Entsprechend besitzt "eine Mutante oder ein Derivat" der zuvor erwähnten Mikroorganismen, die zur Gattung oder Ordnung der Milchsäurebakterien gehören, insbesondere eine Mutante oder ein Derivat der im Rahmen der vorliegenden Anmeldung hinterlegten *Lactobacilli sp.*, und die gleichen Charakteristika wie vorliegend für die beanspruchten Milchsäurebakterien beansprucht, und insbesondere die gleichen wie die hinterlegten Stämme; dies wären zumindest die Fähigkeit zur spezifischen Koaggregation mindestens eines pathogenen Mikroorganismus ausgewählt aus der Gruppe *Staphylococcus aureus* oder *Pseudomonas aeruginosa.* Weiterhin ist bevorzugt mindestens eines der folgenden Merkmale (i) Resistenz der Fähigkeit zur spezifischen Bindung gegenüber einer biologischen, chemischen und/oder physikalischen Behandlung, insbesondere einer Hitzebehandlung bei mehr als 70 °C für mindestens 30 min; (ii) keine Bindung an *Staphylococcus epidermidis* oder weiterer kommensalen Hautkeime; (iii) Fähigkeit zur spezifischen Bindung an zumindest einen pathogenen, Biofilm-bildenden Mikroorganismus; (iv) Fähigkeit zur Hemmung der Biofilmbildung durch *Staphylococcus aureus* oder *Pseudomonas aeruginosa*; (v) Zustandekommen der spezifischen Bindung bei pH 3 - 8. Bevorzugte Derivate können bspw. gentechnologisch hergestellt werden. Dabei beinhaltet der Ausdruck "gentechnologisch hergestellt" im Sinne der Erfindung insbesondere alle Verfahren, die dem Fachmann im Gebiet der Gentechnologie bekannt sind, um Nukleinsäuren *in vitro* und *in vivo* derart zu modifizieren, dass durch rekombinante DNA-Technologien genetische Modifizierungen bewirkt und Gene verändert werden können.

Die erfindungsgemäßen Mikroorganismen liegen dabei vorzugsweise isoliert oder gereinigt vor, wobei der Begriff "isoliert" insbesondere bedeutet, dass die Milchsäurebakterien ihrem Kultivierungsmedium/-milieu - auch bspw. ihrem natürlichen - entnommen sind. Der Ausdruck "gereinigt" ist nicht auf eine absolute Reinheit beschränkt.

Es ist bevorzugt, dass neben den erfindungsgemäßen Mikroorganismen in lebensfähiger Form auch inaktive Formen der erfindungsgemäßen Mikroorganismen vom Rahmen der vorliegenden Erfindung umfasst sind. Dabei bedeutet "inaktive Form" inaktivierte oder tote Zellen, die insbesondere nicht mehr zur Koloniebildung auf Kultivierungsplatten in der Lage sind. Geeignete Verfahren zur Inaktivierung (z. B. biologische, chemische oder physikalische Inaktivierungsmethoden) sind dem Fachmann hinreichend bekannt. Vorliegend können die Mikroorganismen auch in lyophilisierter Form eingesetzt werden. Lyophilisierte Zellen können nach geeigneter Kultivierung in flüssigen oder festen Medium wieder zum anwachsen gebracht werden.

Die Begriffe "inaktivierte Formen" oder "inaktive Form" und "Derivate" oder "Mutanten" schließen vorliegend auch Zell- bzw. Fermentationsüberstände, Lysate, Fraktionen oder Extrakte der erfindungsgemäßen Mikroorganismen mit ein, wobei diese Zell- bzw. Fermentationsüberstände, Lysate, Fraktionen oder Extrakte bevorzugt die Eigenschaften der Milchsäurebakterien besitzen. Hierbei bedeutet "Lysat" - wie auch der Begriff "Extrakt" - insbesondere eine Lösung oder Suspension in einem wässrigem Medium der erfindungsgemäßen Zellen des Mikroorganismus und umfasst bspw. Makromoleküle, wie DNA, RNA, Proteine, Peptide, Lipide, Kohlenhydrate u. ä., sowie insbesondere auch Zelltrümmer. Vorzugsweise umfasst das Lysat auch Zellwand oder Zellwandbestandteile. Verfahren zur Herstellung von Lysaten sind dem Fachmann hinreichend bekannt und umfassen z.B. den Einsatz einer "French Press" oder enzymatische Lyse, Kugelmühle mit Glasperlen oder Eisenkugeln. Das Aufbrechen von Zellen kann enzymatisch, physikalisch oder auch chemisch erfolgen. Beispiele für enzymatische Zelllyse können einzelne Enzyme wie auch Enzymcocktails sein, wie Proteasen, Proteinase K, Lipasen, Glykosidasen; Chemische Lysen können bewirkt werden durch Ionophoren, Detergenzien, wie SDS, Säuren oder Basen; Physikalischen Methoden können durch hohe Drücke, wie French Press, Osmolaritäten, Temperaturen oder Wechsel zwischen Hitze und Kälte bewirkt werden. Ferner können natürlich chemischen, physikalischen und enzymatische Methoden kombiniert werden.

"Inaktivierte Formen" oder "inaktive Form" und "Derivate" oder "Mutanten" der erfindungsgemäßen Mikroorganismen weisen vorzugsweise die gleichen Eigenschaften, wie die benannten Stämme auf. Dabei ist eine metabolische Aktivität bei der "inaktivierten Form" oder "inaktive Form" und "Derivate" vorzugsweise nicht mehr gegeben.

Der Fachmann kann die Begriffe "Derivat" oder "Mutante" mit Inhalt füllen und ohne großen technischen Aufwand die Begriffe im Sinne der vorliegenden Erfindung auslegen. Um Mutanten Zelllysate oder Derivate von den bevorzugten Mikroorganismen bereitzustellen, kann der Fachmann auf Standardliteratur zurückgreifen, die ihm vorliegt und Techniken offenbart, die zur Herstellung von Mutanten Zelllysaten oder Derivaten genutzt werden können.

Mutanten bzw. genetisch veränderte Varianten oder Derivate werden genetisch verändert, beispielsweise durch rekombinante DNA-Technologien (Klonen, Sequenzieren, Transformieren rekombinanter Nukleinsäuren), wie auch physikalische Mutagenese, beispielsweise durch ultraviolette Strahlung, aber auch durch chemische Agenzien, wie mit Ethyl-Methansulfonat (EMS). Dabei können Veränderungen in den positiven Eigenschaften selektiert werdenentweder gezielt oder durch Evaluierung einer Vielzahl von entstandenen Mutanten. Genetisch veränderte Mutanten beinhalten Zellen der erfindungsgemäßen Mikroorganismen und beherbergen rekombinate Nukleinsäuren in ihrem bakteriellen Chromosom und/oder Plasmiden. Modifikationen durch Punktmutationen können zudem Auswirkungen auf die Expression/Transkription/Translation bewirken, wie auch spontane Mutationen ohne direkte genetische Manipulation.

Zellen nach einer Lyophilisation (Gefriertrocknung) sind unter Umständen noch lebensfähig. Diese Zellen können durch spezielle Lagerungsprozesse bei unterschiedlichen Temperaturen inaktiviert werden. Inaktivierte Zellen können z.B. intakte oder aufgebrochene Zellmembranen besitzen, aber keine metabolische Aktivität. Methoden zur Gewinnung von inaktivierten Zellen können sein, z.B. durch eine Behandlung mit Glasperlen, wobei die Einwirkungen der Scherkräfte zwischen Zellen und Glasperlen zum Aufbruch der Zelle führt. Weitere physikalische Methoden, wie French Press, Hochdruckhomogenisation, Kugelmühle oder aber Einfrier-Tau-Prozesse und Autoklavieren führen zur Inaktivierung, wie auch zu Fragmenten der erfindungsgemäßen Mikroorganismen, wie auch UV-Bestrahlung, Autolyseverfahren oder spezielle Lagerungsprozesse bei unterschiedlichen Temperaturen.

Der Begriff "*Lactobacillus*-Zellen" kann im Sinne der Erfindung auch als Milchsäurebakterien oder Lactobazillen bezeichnet werden und umfasst solche Mikroorganismen, die Kohlenhydrate, insbesondere Glukose und Lactose, zur Milchsäurevergärung benötigen und zumeist den Embden-Meyerhof-Biosyntheseweg nutzen. Die Lactobacillus Zellen werden taxonomisch in der Familie Lactobacteriaceae zusammengefasst. Sie sind gram-positiv, nicht sporenbildend und im Allgemeinen unbeweglich. Die Lactobacillus Zellen leben anaerob, sind jedoch aerotolerant, obwohl sie keine Hämine (Cytochrom, Katalase) enthalten (Schleifer et al., System. Appl. Microb.: 18, 461-467 (1995) oder Ludwiq et al., System. Appl. Microb. 15: 487-501 (1992). Die *Lactobacillus*-Zellen bzw. die Spezies können auf der Grundlage des Kohlenhydratverwertungsmusters bestimmt werden, insbesondere mittels dem API Test (Firma biomerieux) oder auch über 16sRNA Sequenzierung. Erfindungsgemäß umfasst sind insbesondere solche Spezies, die für eine homofermentative Milchsäuregärung oder heterofermentative Milchsäuregärung geeignet sind. Die erwähnten DSMZ-Hinterlegungen wurden gemäß dem Budapester Vertrag über die internationale Anerkennung der Hinterlegung von Mikroorganismen zum Zwecke der Patenthinterlegung vorgenommen.

Wie weiter oben erwähnt, betrifft die vorliegende Erfindung auch Zusammensetzungen, enthaltend bevorzugt lebensfähige Mikroorganismen oder ein Zelllysat Mutante oder Derivat davon, sowie bevorzugt zumindest einen Träger oder Exzipienten, der ausgewählt ist aus zumindest einem der folgenden: einen kosmetisch verträglichen Träger oder Exzipienten, einem pharmazeutisch verträglichen Träger oder Exzipienten oder einem dermatologisch verträglichen Träger oder Exzipienten.

Dabei wird unter einer "Zusammensetzung" im Sinne der vorliegenden Erfindung insbesondere jede Zusammensetzung verstanden, die zumindest einen erfindungsgemäßen Mikroorganismus aufweist, oder ein Derivat Zelllysat oder eine Mutante davon, sowie ggf. weitere Inhaltsstoffe wie Träger oder Hilfsstoffe, oder ggf. noch weitere aktive Inhaltsstoffe und Salze. Unter kosmetisch, pharmazeutisch oder dermatologisch verträglichen Hilfsstoffen, Trägern oder Exzipienten wird dabei jede Substanz oder Stoff verstanden, der jeweils üblicherweise im kosmetischen, pharmazeutischen oder im Dentalbereich eingesetzt wird, um einen aktiven Inhaltsstoff der Zusammensetzung, vorliegend also der zumindest eine Mikroorganismus Zelllysatoder Derivat oder Mutante davon, jeweils kosmetisch oder pharmazeutisch einzusetzen, zu verabreichen und zur Wirkung zu bringen.

Es ist bevorzugt, dass die Zusammensetzung nicht nur einen der erfindungsgemäßen Mikroorganismen oder Zelllysat, Mutante oder Derivat davon aufweisen kann, sondern auch eine Mischung von erfindungsgemäßen Mikroorganismen oder eine Mischung der Zelllysate, Derivate oder Mutanten oder aber eine Mischung aus den erfindungsgemäßen Mikroorganismen und den Zelllysaten, Derivaten oder Mutanten.

Die Zusammensetzung kann bevorzugt in fester oder flüssiger oder viskoser Form oder als Aerosol vorliegen, und kann u.a. in Form von Pulvern, Tabletten, Lösungen, Granulaten, Suspensionen, Emulsionen, Kapseln, Pasten, Gelen, Sprays, etc. verwendet werden, mithin in jeder Form, die für eine Verabreichung geeignet ist. Ferner ist bevorzugt, dass die Zusammensetzung weitere Probiotika, Antiseptika oder andere antibakteriell wirksame Substanzen umfasst, sowie bevorzugt ggf. Saccharide und Konservierungsmittel, Geschmacksstoffe, Süßstoffe, Vitamine, Mineralien, etc. Die Druckschrift EP 2 133 414 A1 listet eine Reihe von Inhaltsstoffen auf, die für derartige Zusammensetzungen eingesetzt werden können; auf diese Veröffentlichung wird explizit Bezug genommen, so dass sie zum Offenbarungsgehalt der vorliegenden Anmeldung zu rechnen ist. Außerdem können Füllstoffe, Fließmittel, Rheologiemodifikatoren, Weichmacher, Stabilisatoren, Initiatoren oder auch reaktiv vernetzende Monomere wie Methacrylate o.ä. in der Zusammensetzungen vorhanden sein.

Die erfindungsgemäße Zusammensetzung wird dabei insbesondere zur Verwendung in der Körperhygiene, -therapie und -prophylaxe eingesetzt, und weist zumindest einen erfindungsgemäßen Mikroorganismus oder Derivate, Mutante oder Zelllysate davon auf.

Die Zusammensetzung bzw. der Mikroorganismus kann ferner auch als Desinfektionsmittel, bspw. als Flächendesinfektion, wie insbesondere auch als Reinigungslöung für Kontaktlinsen eingesetzt werden.

Die Dosierung und die Verabreichung an sich, mit welcher die erfindungsgemäße Zusammensetzung eingesetzt wird, wird vom jeweiligen Einsatz und vom jeweiligen Patienten - insbesondere von dessen Alter, Gewicht, dem allgemeinen Zustand, etc. - abhängen und liegt im Können und der Einschätzung des Fachmanns, der die Zusammensetzung zum Einsatz bringt. Dabei kann die erfindungsgemäße Zusammensetzung kosmetisch, als Medizinprodukt vorliegen oder pharmazeutisch sein. Vorzugsweise umfasst die Zusammensetzung die Milchsäurebakterien in einer Menge mit einem Masseanteil von 0,001 Gewichtsprozent bis 10 Gewichtsprozent, bevorzugt bis 0,005 Gewichtsprozent bis 5 Gewichtsprozent, besonders bevorzugt 0,01 Gewichtsprozent bis 3 Gewichtsprozent vorliegt. Es war völlig überraschend, dass die Verwendung eines Masseanteils von 0,001 Gewichtsprozent bis 10 Gewichtsprozent insbesondere dazu führte, dass die Zusammensetzung länger Nutzbar, d. h. haltbar war. Wenn die Milchsäurebakterien in einer Menge mit einem Masseanteil von 0,005 Gewichtsprozent bis 5 Gewichtsprozent verwendet wurden, führte dies überraschenderweise dazu, dass sich dies positiv auf die rheologischen Eigenschaften der Zusammensetzung auswirkten und diese z. B. eine geringere Viskosität aufweist und somit besser auf der Haut zu verteilen ist. Eine Verwendung der Milchsäurebakterien in einer Menge mit einem Masseanteil von 0,01 Gewichtsprozent bis 3 Gewichtsprozent hat überraschenderweise dazu geführt, dass sich die Komponenten der Zusammensetzung besser miteinander verbinden und in einer verringerten Arbeitszeit eine homogene Zusammensetzung bereitgestellt werden kann, was wiederum zur Reduzierung der Herstellungskosten führt. Es versteht sich, dass für spezifische Anwendungen aber auch andere Mengen eingesetzt werden können, die sich von den hierin angegebenen unterscheiden.

Wie weiter oben erwähnt, kann gemäß einer bevorzugten Ausführungsform die Zusammensetzung bzw. der Mikroorganismus im Bereich der Körperhygiene eingesetzt werden, um die genannten pathogenen Keime zu aggregieren, d.h. zu binden. Diese Koaggregate können dann leicht entfernt, bspw. bei einer Suspension insbesondere ausgespült werden, wodurch vorteilhafterweise eine Reduktion der pathogenen Keime erzielt wird. Die Milchsäurebakterien bzw. eine diese enthaltende Zusammensetzung kann hierzu unterschiedlich eingesetzt werden. Bspw. können sie in Duschgelen, Duschlotionen, Körperlotionen, Flüssigseifen, Seifen, Duschöle aber auch in Desinfektionslösungen, Filtersystemen in Beatmungsgeräte, Nasensprays, Reinigungslösung für Kontaktlinsen, Handtücher oder Reinigungstücher angewendet werden. Daher betrifft die vorliegende Erfindung auch sämtliche Produkte, die im Bereich der Körperhygiene und Medizinprodukte und Prophylaxe eingesetzt werden und die die erfindungsgemäßen Milchsäurebakterien enthalten. Oben beschriebene Ausführungsformen gelten entsprechend für den Bereich der Behandlung, Therapie und Prophylaxe bei Säugetieren. Die Mikroorganismen bzw. eine diese enthaltende Zusammensetzung kann hierzu unterschiedlich eingesetzt werden.

Insbesondere ist bevorzugt, wenn die erfindungsgemäße Zusammensetzung und/oder der erfindungsgemäße Mikroorganismus zur Herstellung eines Arzneimittels, zur Behandlung oder Prophylaxe von Diabetes mellitus, Hauterkrankungen, Hautverletzungen, Toxic-Shock-Syndrom, das *Staphylococcal Scaled Skin Syndrom,* sowie die Toxi-Infektion durch Enterotoxine, Furunkel, Karbunkel, Sinusitis, Osteomyelitis, wie die Sepsis und im Gefolge eine Meningitis und/oder Herzmuskel- sowie Herzbeutelentzündung (Myo- und Pericarditis) durch *Staphylococcus aureus* und Pneumonien bei zystischer Fibrose, Harnwegsinfekte, Enterokolitis, Meningitis, Otitis externa, Infektionen auf Brandwunden durch *Pseudomonas aeruginosa* verwendet wird.

Durch die erfindungsgemäßen Mikroorganismen und die diese enthaltende Zusammensetzung werden Mittel bereit gestellt, mit welchen diese Erkrankungen vorteilhaft behandelt, bzw. vermieden werden können. Die Mikroorganismen bzw. die diese enthaltenden Zusammensetzungen können dabei sowohl in der Human- als auch in der Veterinärmedizin, insbesondere, wie bereits aufgeführt, bei Hund, Affe, Katze, Pferd und Nagetiere (Hasen, Kaninchen, Hamster, Meerschweinchen) und Nutztiere, wie Huhn, Schwein und Rind, Schaf, Ziege, u.a. Haus- und Nutztieren eingesetzt werden.

Die erfindungsgemäße Zusammensetzung bzw. die Mikroorganismen - oder Zelllysate Derivate oder Mutanten davon - können insbesondere als Nahrungsmittelzusatz, als Hygieneprodukt, bzw. als ein die Mikroorganismen aufweisenden Hygieneprodukts, oder als eine pharmazeutische Präparation eingesetzt werden. Solche Hygieneprodukte können bspw. auch Kits sein, die neben Körperhygienischen Vorrichtungen oder Geräten, Spülungen, Pasten u.ä. auch die erfindungsgemäßen Mikroorganismen oder die diese enthaltenden Zusammensetzungen enthalten.

Es versteht sich, dass die vorstehend genannten und nachstehend noch zu erläuternden Merkmale nicht nur in der jeweils angegebenen Kombination, sondern auch in Alleinstellung verwendbar sind, ohne den Rahmen der vorliegenden Erfindung zu verlassen.

Die anmeldungsgemäße Lehre zeichnet sich durch folgende Merkmale aus:

- Abkehr vom technisch Üblichen
- neue Aufgabenstellung
- Vorliegen eines seit langem ungelösten dringenden Bedürfnisses für die Lösung des mit der Erfindung gelösten Problems
- bisheriges vergebliches Bemühen der Fachwelt
- die Einfachheit der Lösung spricht für erfinderische Tätigkeit, insbesondere da sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteiltechnischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren Mittels, Eröffnung eines zweiten Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- glücklicher Griff, da aus einer Vielzahl von Möglichkeiten eine bestimmte gewählt wurde, deren Ergebnis nicht vorausgesagt werden konnte, daher handelt es sich um ein patentwürdigen glücklichen Griff
- Irrtümer in der Fachliteratur bzw. sehr widersprüchliche Darstellung zum Erfindungsgegenstand
- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

Insbesondere die vorteilhaften Ausführungsformen der Erfindung weisen mindestens einen oder mehrere der genannten Vorteile auf.

## BESCHREIBUNG DER FIGUREN

Im Folgenden soll die Erfindung beispielhaft anhand von Figuren und Beispielen erläutert werden, ohne jedoch hierauf beschränkt zu sein. Es zeigen:

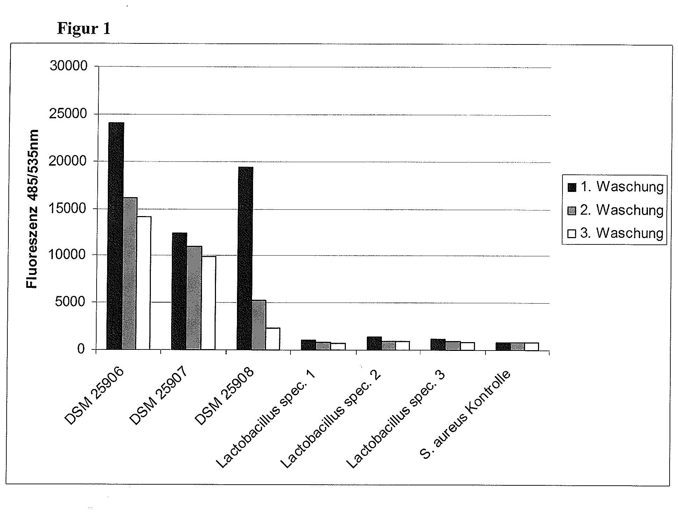

Fig. 1: Die spezifische Bindung bzw. Aggregation eines Ausführungsbeispiels der erfindungsgemäßen Mikroorganismen (DSM 25906, DSM 25907, DSM 25908) an durch *Staphylococcus aureus* ausgebildeten Biofilm (Bindungs-Assay) nach 3 maligen Auswaschen der nicht-gebundenen Zellen sowie im Vergleich dazu weitere, nicht erfindungsgemäße *Lactobacillus*-Stämme, die nicht zur Bindung an *Staphylococcus aureus* befähigt sind (Lactobacillus spec.1 und Lactobacillus spec.2 und Lactobacillus spec.3); spezifische Quantifizierung der Bindung bzw. Aggregation von CFDA-markierten erfindungsgemäßem *Lactobacillus* an den Biofilm in 96-Well-Mikrotiterplatte über Fluoreszenzmessung (485/535nm).

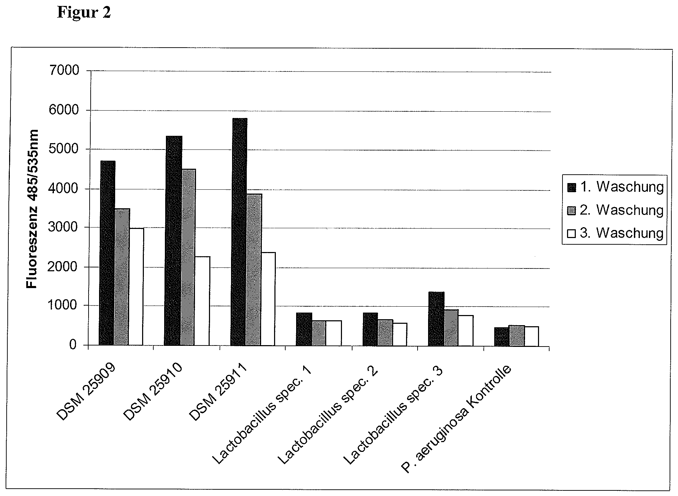

Fig. 2: Die spezifische Bindung bzw. Aggregation eines Ausführungsbeispiels der erfindungsgemäßen Mikroorganismen (DSM 25909, DSM 25910, DSM 25911) an durch *Pseudomonas aeruginosa* ausgebildeten Biofilm (Bindungs-Assay) nach 3 maligen Auswaschen der nicht-gebundenen Zellen sowie im Vergleich dazu weitere, nicht erfindungsgemäße *Lactobacillus* Stämme, die nicht zur Bindung an *Pseudomonas aeruginosa* befähigt sind (Lactobacillus spec.1 und Lactobacillus spec.2 und Lactobacillus spec.3); spezifische Quantifizierung der Bindung bzw. Aggregation von CFDA-markierten erfindungsgemäßem Lactobacillus an den Biofilm in 96-Well-Mikrotiterplatte über Fluoreszenzmessung (485/535nm).

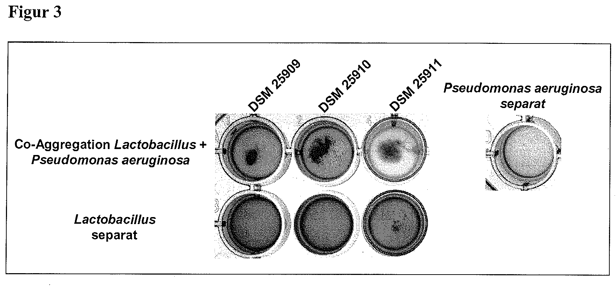

Fig. 3: Die makroskopische Kontrolle der spezifischen Bindung eines Ausführungsbeispiels eines erfindungsgemäßen Mikroorganismus ((DSM 25909, DSM 25910, DSM 25911) an *Pseudomonas aeruginosa* Zellen nach der Co-Aggregation, sowie die erfindungsgemäßen *Lactobacillus*-Stämme und der Targetstamm separat in einer Photodokumentationsanlage.

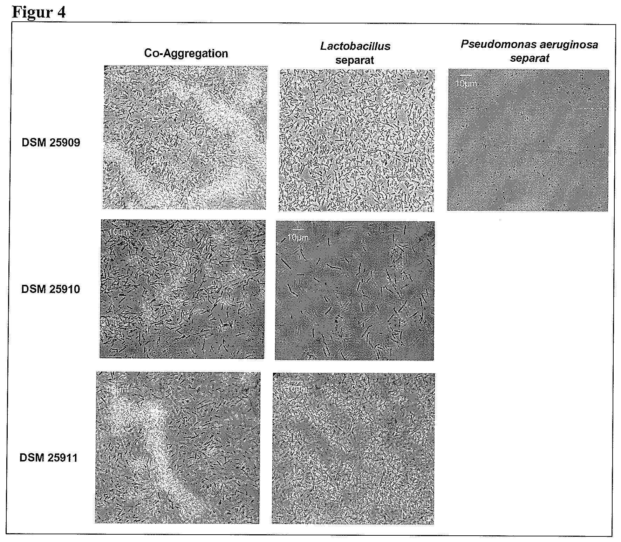

Fig. 4: Die mikroskopische Kontrolle der spezifischen Bindung eines Ausführungsbeispiels eines erfindungsgemäßen Mikroorganismus (DSM 25909, DSM 25910, DSM 25911) an *Pseudomonas aeruginosa* Zellen nach der Co-Aggregation, sowie die erfindungsgemäßen Lactobacillus-Stämme und der Targetstamm separat. Mikroskopische Abbildung (Phasenkontrast, 1000x vergrößert).

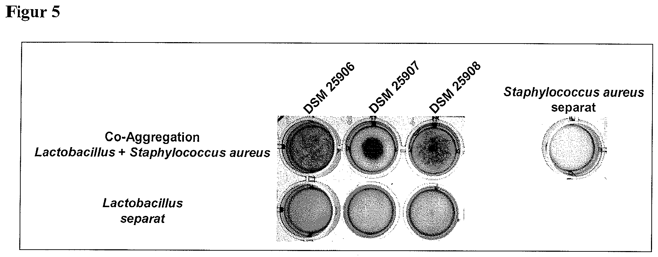

Fig. 5: Die makroskopische Kontrolle der spezifischen Bindung eines Ausführungsbeispiels eines erfindungsgemäßen Mikroorganismus (DSM 25906, DSM 25907, DSM 25908) an *Staphylococcus aureus* Zellen nach der Co-Aggregation, sowie die erfindungsgemäßen Lactobacillus-Stämme und der Targetstamm separat in einer Photodokumentationsanlage.

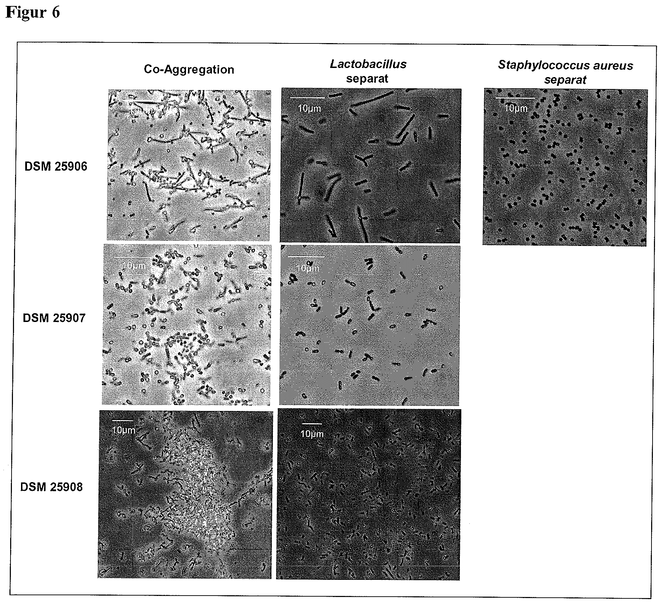

Fig. 6: Die mikroskopische Kontrolle der spezifischen Bindung eines Ausführungsbeispiels eines erfindungsgemäßen Mikroorganismus (DSM 25906, DSM 25907, DSM 25908) an *Staphylococcus aureus* Zellen nach der Co-Aggregation, sowie die erfindungsgemäßen Lactobacillus-Stämme und der Targetstamm separat. Mikroskopische Abbildung (Phasenkontrast, 1000x vergrößert).

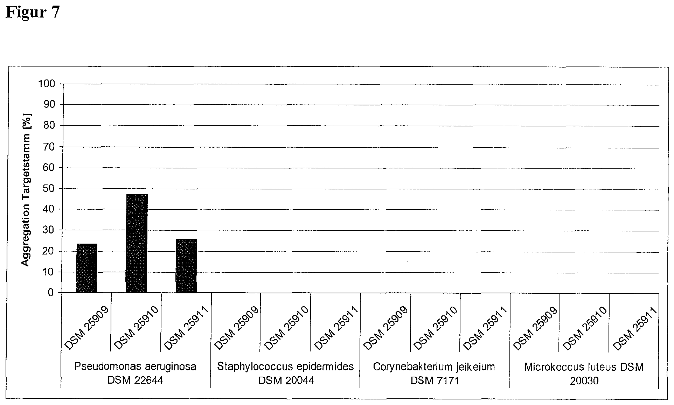

Fig. 7: Die spezifische Aggregation eines Ausführungsbeispiels der erfindungsgemäßen Mikroorganismen (DSM 25909, DSM 25910, DSM 25911) von *Pseudomonas aeruginosa* (DSM 22644), *Staphylococcus epidermides* (DSM 20044), *Corynebakterium jeikeium* (DSM 7171) und *Micrococcus luteus* (DSM 20030) im Co-Aggregationsassay; spezifische Quantifizierung der Aggregation durch Messung der optischen Dichte bei 600nm des Targetkeims separat, sowie die der Lactobacillus-Stämme separat und die des Co-Aggregationsansatzes.

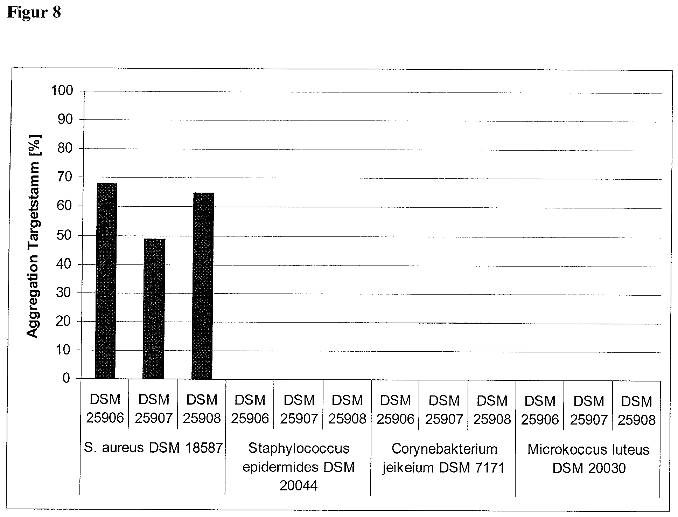

Fig. 8: Die spezifische Aggregation eines Ausführungsbeispiels der erfindungsgemäßen Mikroorganismen (DSM 25906, DSM 25907, DSM 25908) von *Staphylococcus aureus* (DSM 18587), *Staphylococcus epidermides* (DSM 20044), *Corynebakterium jeikeium* (DSM 7171) und *Micrococcus luteus* (DSM 20030) im Co-Aggregationsassay; spezifische Quantifizierung der Aggregation durch Messung der optischen Dichte bei 600nm des Targetkeims separat, sowie die der Lactobacillus-Stämme separat und die des Co-Aggregationsansatzes. Im Sinne der Erfindung wird der Begriff "Co-Aggregation" bzw. "co-aggregieren" synonym zu "Koaggregation" bzw. "koaggregieren" verwendet.

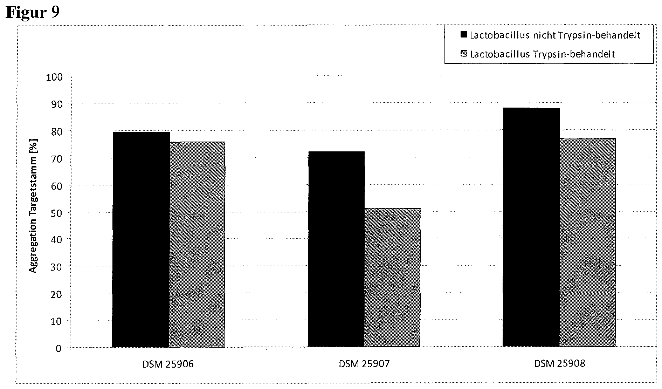

Fig. 9: Die spezifische Aggregation eines Ausführungsbeispiels der erfindungsgemäßen Mikroorganismen (DSM 25906, DSM 25907, DSM 25908) mit und ohne Trypsin-Behandlung von *Staphylococcus aureus* (DSM 18587) im Co-Aggregationsassay; spezifische Quantifizierung der Aggregation durch Messung der optischen Dichte bei 600nm des Targetkeims separat, sowie die der *Lactobacillus*-Stämme separat und die des Co-Aggregationsansatzes.

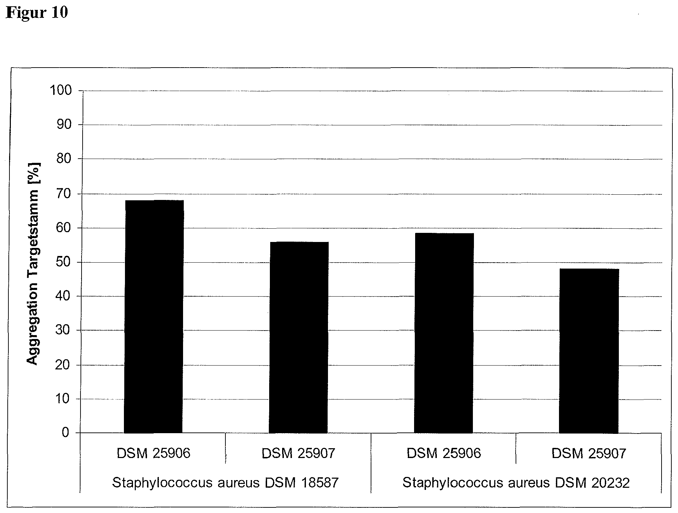

Fig. 10: Die spezifische Aggregation eines Ausführungsbeispiels der erfindungsgemäßen Mikroorganismen (DSM 25906 und DSM 25207) von *Staphylococcus aureus* (DSM 18587) und *Staphylococcus aureus* (DSM 20232) im Co-Aggregationsassay; spezifische Quantifizierung der Aggregation durch Messung der optischen Dichte bei 600nm des Targetkeims separat, sowie die der *Lactobacillus*-Stämme separat und die des Co-Aggregationsansatzes.

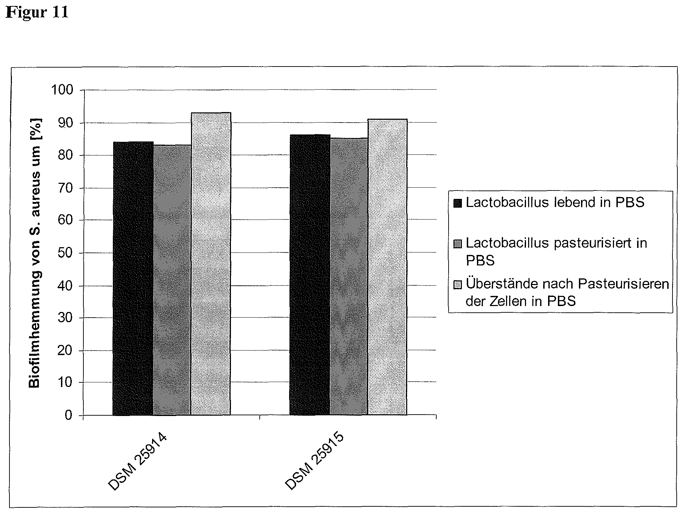

Fig. 11: Die Hemmung der Biofilmbildung eines Ausführungsbeispiels der erfindungsgemäßen Mikroorganismen (DSM 25914 und DSM 25915) des durch *Staphylococcus aureus* ausgebildeten Biofilm nach 6stündiger Inkubation durch in PBS gewaschene *Lactobacillus*-Suspension, hitze-inaktivierte *Lactobacillus*-Suspension und Überstände der hitze-inaktivierten *Lactobacillus*-Suspension; Quantifizierung des Biofilms durch KristallviolettFärbung in 96-Well-Mikrotiterplatte über Messung der optischen Dichte bei 590nm.

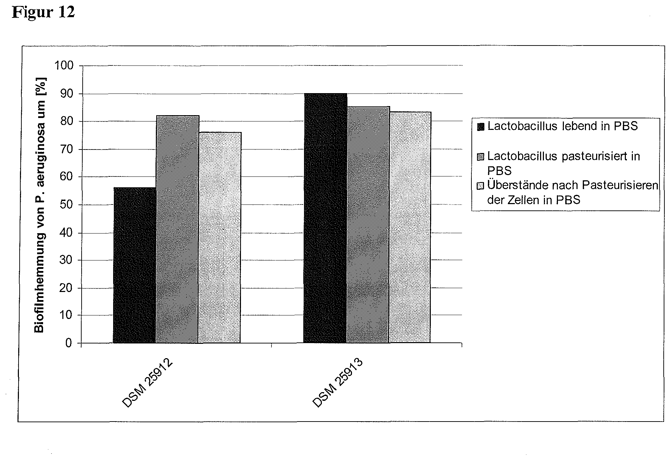

Fig. 12: Die Hemmung der Biofilmbildung eines Ausführungsbeispiels der erfindungsgemäßen Mikroorganismen (DSM 25912 und DSM 25913) des durch *Pseudomonas aeruginosa* ausgebildeten Biofilm nach 6stündiger Inkubation durch in PBS gewaschene *Lactobacillus*-Suspension, hitze-inaktivierte *Lactobacillus*-Suspension und Überstände der hitze-inaktivierten *Lactobacillus*-Suspension; Quantifizierung des Biofilms durch KristallviolettFärbung in 96-Well-Mikrotiterplatte über Messung der optischen Dichte bei 590nm.

**DETAILLIERTE BESCHREIBUNG DER AUSFÜHRUNGSBESPIELE**

**Beispiel 1**

Zur Identifizierung und Selektion von erfindungsgemäßen Mikroorganismen wurden aus einer *Lactobacillus*-Datenbank verschiedene Stämme durch ein vier-stufiges Screening untersucht, wobei zunächst hinsichtlich der Bindungsfähigkeit an pathogene Hautkeime (im Folgenden auch "Targetkeime") gescreent wurde (Bindungs-Assay), und anschließend die im ersten Schritt identifizierten Stämme in einem Co-Aggregationsassay im Mikrotiterplatten-Maßstab geprüft wurden, wobei die Co-Aggregation mit dem jeweiligen Targetkeim qualitativ mittels binokularen Stereomikroskops nachgewiesen wurde. Ferner wurde die Stärke der Co-Aggregation und die Stabilität der Bindung mit dem Targetkeim, sowie die Fähigkeit zur Biofilmverhinderung untersucht, was schließlich zu den identifizierten beispielhaften erfindungsgemäßen Mikroorganismen (*Lactobacillus)* führte.

Bindungs-Assay

Um die Bindungsaktivität selektierter *Lactobacillus* Stämme quantifizieren zu können, wurde ein *Bindungs-Assay* etabliert, welcher den quantitativen Nachweis der Bindung der Lactobacillus Stämme an hautpathogene Mikroorganismen in einer 96-Well-Platte ermöglichte. Die Bindungsaktivität der *Lactobacillus* Zellen mit den hautpathogenen Keimen korreliert mit der Co-Aggregationsaktivität bzw. Co-Aggregationsfähigkeit. Dies wurde experimentell überprüft. Dazu zeigt Fig. 2 exemplarisch die Bindung des *Lactobacillus* DSM 25909, DSM 25910, DSM 25911 und *Pseudomonas aeruginosa* DSM 22644, sowie Fig. 1 *Lactobacillus* DSM 25906, DSM 25907, DSM 25908 und *Staphylococcus aureus* DSM 22644.

Die Messmethode basiert auf der spezifischen Bindung der erfindungsgemäßen Stämme an in einem Biofilm gebundene Targetstämme. Für den Assay werden definierte Mengen der erfindungsgemäßen Stämme mit einem Fluoreszenzfarbstoff (CFDA-Lösung, Invitrogen) markiert und mit einer definierten Menge an in einem Biofilm gebundenen Targetkeimen gemischt. Der Verbleib der gebunden, erfindungsgemäßen Stämme an dem Targetstamm wird nach mehrmaligen Waschen mittels Fluoreszenzphotometer vermessen.

Zur Durchführung dieser Versuche wurde der Targetkeim *Pseudomonas aeruginosa* (DSMZ 22644) und *Staphylococcus aureus* (DSMZ 18587) nach Standardprotokollen kultiviert. Dafür wurde TSB (Trypticase Soy Broth) für *Pseudomonas aeruginosa* und TSY (Trypticase Soy Yeast Extract Medium) *Staphylococcus aureus* verwendet.

Die *Lactobacillus*-Stämme wurden in MRS-Medium (de Man *et al.*, 1960) anaerob bei 37 °C kultiviert.

Zur Aufarbeitung der Targetkeime wurden die Zellen jeweils nach Erreichen der beginnenden stationären Wachstumsphase geerntet, 3 Mal mit Phosphat-gepufferter Saline (PBS, pH 7,4) gewaschen und in PBS aufgenommen. Dann wurden 100 µl der Suspension pro Well in eine 96-Well-Mikrotiterplatte vorlegt.

Während einer 6stündigen anaeroben Inkubation kommt es zur Ausbildung eines Biofilmes durch die Targetstämme. Nach der Inkubation werden nicht gebundene Zellen durch 3 maligen Waschen mit Phosphat-gepufferter Saline (PBS, pH 7,4) entfernt.

Zur Aufarbeitung der *Lactobacillus*-Stämme wurden diese nach 24stündiger Kultivierung geerntet, 3 Mal mit PBS gewaschen, in PBS aufgenommen und durch Zugabe der CFDA-Lösung (Invitrogen) fluoreszenzmarkiert.

Zur Durchführung des Bindungs-Assays wurden zu den in einem Biofilm gebundenen Targetstämmen pro Well 100µl der *Lactobacillus*-Suspension hinzugefügt. Parallel erfolgten Kontrollansätze ohne Zugabe der *Lactobacillen*-Suspension. Nach 1 stündiger Inkubation bei 30 °C in einem Brutschrank wurden die nicht gebundenen Zellen abgetrennt und 3 Mal mit PBS gewaschen. Nach jedem Waschschritt wurde die Fluoreszenz im Fluoreszenzplattenphotometer (Em485nm/Ex535nm) gemessen.

Die Zunahme der Fluoreszenz (Em 485 nm/Ex 535 nm) im Ansatz mit *Lactobacillus* Stämmen im Vergleich zur Kontrolle ohne *Lactobacillus*-Zellen korrelierte dabei mit der Menge an Biofilm-gebundenen *Lactobacillus*-Zellen, Die gemessene Fluoreszenz nach Bindung der *Lactobacillus*-Zellen entsprach der Bindungsstärke. Je größer dieser Wert war, desto besser banden die markierten *Lactobacillus*-Zellen an die im Biofilm gebundenen Targetstämme. Und desto stärker ist die Bindungsaktivität der untersuchten *Lactobacillus*-Zellen.

Die Versuche ergaben, dass die selektierten *Lactobacillus*-Stämme für den Targetkeim/Targetstamm *Pseudomonas aeruginosa bzw. Staphylococcus aureus* im *Bindungs-Assay* nach Entfernung der ungebundenen Zellen zu einer 3 bis 17-fachen Erhöhung der Fluoreszenz infolge der Bindung der markierten *Lactobacillus*-Zellen an die im Biofilm gebundenen Targetstämme nach 3 Waschungen führte. Beispielhaft für die erfindungsgemäßen Mikroorganismen sind in Figur 2 die Ergebnisse des Bindungs-Assays für *Lactobacillus* DSM 25909, DSM 25910 und DSM 25911mit *Pseudomonas aeruginosa* und DSM 25906, DSM 25907 und DSM 25908 mit *Staphylococcus aureus* in Figur 1 dargestellt. Weiter sind in Figur 1 und 2 zum Vergleich die Daten nicht erfindungsgemäßer *Lactobacillus*-Stämme dargestellt (Lactobacillus spec. 1 und 2), welche nicht zur spezifischen Bindung an die Targetkeime befähigt sind.

**Beispiel 2**

Co-Aggregationsassay

Um die Co-Aggregationsaktivität selektierter *Lactobacillus* Stämme zu veranschaulichen, dient der nachfolgende Nachweis im 0,8ml-Volumen bzw. in einer 24-*Well*-Platte.

Bei dieser Methode wird die Aggregationsverhalten der *Lactobacillen*, sowohl des Targetkeims separat betrachtet und schließlich die Co-Aggregation, Lactobacillus und Targetstamm zusammen im Gemisch. Die Betrachtung erfolgt makroskopisch durch Fotographie die 24-*Well*-Platte, wie auch mikroskopisch.

Zur Durchführung dieser Versuche wurde der Targetkeim *Pseudomonas aeruginosa* (DSMZ 22644) und *Staphylococcus aureus* (DSMZ 18587) nach Standardprotokollen kultiviert. Dafür wurde TSB (Trypticase Soy Broth) für *Pseudomonas aeruginosa* und TSY (Trypticase Soy Yeast Extract Medium) verwendet.

Die *Lactobacillus*-Stämme wurden in MRS-Medium (de Man *et al.*, 1960) anaerob bei 37 °C kultiviert.

Zur Aufarbeitung der Targetkeime wurden die Zellen nach 16h geerntet, 3 Mal mit Phosphat-gepufferter Saline (PBS, pH 7,4) gewaschen und auf die $OD_{600}$=4 justiert.

Zur Aufarbeitung der *Lactobacillus*-Stämme wurden diese nach 16h geerntet, 2 Mal mit PBS gewaschen und anschließend in PBS-Volumen aufgenommen und entsprechend auf eine $OD_{600}$=8 justiert.

Zur Durchführung des Co-Aggregationsassays wurden je 400 µl markierte Targetkeim-Suspension pro Well mit 400 µl *Lactobacillus*-Suspension in einer 24-Well-Platte vermengt. Parallel erfolgten Kontrollansätze mit 400 µl des jeweiligen Targetkeims plus 400 µl PBS (Kontrolle 1), oder 400 µl der jeweiligen *Lactobacillus*-Suspension plus 400 µl PBS (Kontrolle 2). Nach 30 minütiger Inkubation bei 25 °C auf einem Tischschüttler wurden die Ansätze makroskopisch mittels Photodokumentationsanlage, wie auch mikroskopisch, indem 3µl aus der Mitte der Wells auf einen Objektträger überführt wurden, betrachtet.

Zur Quantifizierung der Co-Aggregation wurden die entstehenden Co-Aggregate durch Zentrifugation (10 sec, 300xg) abgetrennt. Anschließend wurden 100 µl vom Überstand in eine 96-Well-Platte mit Flachboden überführt und die optische Dichte bei 600nm im Plattenphotometer gemessen.

Zur prozentualen Berechnung der Co-Aggregationsaktivität wurde die folgende Formel zu Rate gezogen:

$$\%Aggregation = \frac{(OD_{T\arg etstamm\,separat} + OD_{Lactobacillus\,separat}) - OD_{Co-Aggregation}}{OD_{T\arg etstamm\,separat} + OD_{Lactobacillus\,separat}} x100$$

Dabei wurden die optische Dichte des Targetkeims, sowie die der *Lactobacillus*-Stämme und die des Co-Aggregationsansatzes für die Berechnung der prozentualen Co-Aggregationsaktivität zu Rate gezogen.

Die Versuche zeigen, dass die selektierten *Lactobacillus*-Stämme für den Targetkeim/Targetstamm *Staphylococcus aureus* bzw. *Pseudomonas aeruginosa* in der Co-Aggregation, es zu Verklumpungen (Aggregaten) im Gemisch resultiert, welche makroskopisch durch dunkle Bereiche im Well sichtbar sind. In den Wells, welche die Targetstämme bzw. die erfindungsgemäßen *Lactobacillus*-Stämme separat enthält kommt es zu keiner Aggreagatbildung. Dies ist sichtbar durch keine Bildung dunkler Bereiche im Well. Die makroskopischen Resultate im Co-Aggregationsansatz sind für die selektierten *Lactobacillus* Stämme für den Targetkeim/Targetstamm *Staphylococcus aureus* in Figur 5 bzw. *Pseudomonas aeruginosa* in Figur 3 dargestellt. In der mikroskopischen Betrachtung zeigen sich durch alle erfindungsgemäßen *Lactobacillus* Stämme eindeutige Affinitäten zu den jeweiligen Targetstämmen, welches zu unterschiedlichen Aggegatgrößen in der mikroskopischen Betrachtung der Co-Aggregation führt. Die mikroskopischen Resultate im Co-Aggregationsansatz sind für die selektierten *Lactobacillus*-Stämme für den Targetkeim/Targetstamm *Staphylococcus aureus* in Figur 6 bzw. *Pseudomonas aeruginosa* in Figur 4 dargestellt.

**Beispiel 3**

Ferner wurden Versuche durchgeführt, um den Einfluss von Proteasen auf die erfindungsgemäßen *Lactobacillus*-Stämme und deren Aggregationseigenschaften auf die Targetkeime zu zeigen.

Dafür wurden die erfindungsgemäßen Lactobacillus-Stämme und die Targetkeime, wie in Beispiel 2 beschrieben, bearbeitet.

Die erfindungsgemäßen Lactobacillus-Stämme wurden protease-behandelt, hier beschrieben am Beispiel der Protease, Trypsin. Nach der Bearbeitung der *Lactobacillus*-Stämme erfolgte die Behandlung mit Trypsin (12,4 Units/mg, Sigma) bei 37°C für 60min behandelt. Anschließend wurden Zellen erneut eine 2 Mal mit PBS gewaschen und die Aggregationseigenschaften quantifiziert, wie in Beispiel 2 beschrieben.

Die Versuche ergaben, dass nach Protease-Behandlung die erfindungsgemäßen *Lactobacillus*-Stämme für den Targetkeim/Targetstamm *Pseudomonas aeruginosa* bzw. *Staphylococcus aureus* die Aggregationseigenschaften im Co-Aggregationsassay zu einer 50 - 76%igen Aggregation des Targetkeims. Beispielhaft für die erfindungsgemäßen Mikroorganismen sind in Figur 9 die Ergebnisse des Co-Aggregationsassays für Lactobacillus DSM 25906, DSM 25907 und DSM 25908 mit und ohne Trypsin-Behandlung mit *Staphylococcus aureus* dargestellt.

**Beispiel 4**

Des Weiteren wurden Versuche mit weiteren kommensalen Hautkeimen durchgeführt, um die Spezifität der Aggregationseigenschaften der erfindungsgemäßen *Lactobacillus*-Stämme gegen die Targetkeime darzustellen. Dafür wurden *Staphylococcus epidermides* (DSM 20044), *Corynebakterium jeikeium* (DSM 7171) und *Micrococcus luteus* (DSM 20030) ausgewählt. Dafür wurden die erfindungsgemäßen Lactobacillus-Stämme und die Targetkeime, wie in Beispiel 2 beschrieben, kultiviert und aufgearbeitet.

Zur Durchführung dieser Versuche wurde alle zu untersuchenden Targetkeim *Pseudomonas aeruginosa* (DSMZ 22644), *Staphylococcus aureus* (DSMZ 18587) *Staphylococcus epidermides* (DSM 20044), *Corynebakterium jeikeium* (DSM 7171) und *Micrococcus luteus* (DSM 20030) nach Standardprotokollen kultiviert. Dafür wurde TSB (Trypticase Soy Broth) für *Pseudomonas aeruginosa* und TSY (Trypticase Soy Yeast Extract Medium) für *Staphylococcus aureus* (DSMZ 18587), *Staphylococcus epidermides* (DSM 20044), *Corynebakterium jeikeium* (DSM 7171) und *Micrococcus luteus* (DSM 20030) verwendet.

Die Aufarbeitung der Targetstämme und der C-Aggregationsassay erfolgte wie im Beispiel 2 beschrieben.

Die Versuche ergaben, dass es zu keiner Aggregation gegen die weiteren untersuchten Targetstämme durch die erfindungsgemäßen *Lactobacillus*-Stämme, sondern es sich um sehr spezifische Eigenschaften handelt. Beispielhaft für die erfindungsgemäßen Mikroorganismen sind in Figur 8 die Ergebnisse des Co-Aggregationsassays für Lactobacillus DSM 25906, DSM 25907 und DSM 25908 mit *Staphylococcus aureus* und in Figur 7 für Lactobacillus DSM 25909, DSM 25910 und DSM 25911 mit *Pseudomonas aeruginosa* dargestellt.

**Beispiel 5**

Ferner wurden Versuche mit weiteren Keimen der gleichen Gattung und Spezies Hautkeimen durchgeführt, um die Spezifität der Bindungs- bzw. Aggregationseigenschaften der erfindungsgemäßen *Lactobacillus*-Stämme gegen die Targetkeime der gleichen Gattung und Spezies darzustellen. Dafür wurde *Staphylococcus aureus* (DSMZ 20232) ausgewählt um deren Aggregationseigenschaften auf die Targetkeime zu zeigen.

Dafür wurden die erfindungsgemäßen Lactobacillus-Stämme und die Targetkeime, wie in Beispiel 2 beschrieben, kultiviert und aufgearbeitet.

Zur Durchführung dieser Versuche wurde alle zu untersuchenden Targetkeime, in diesem Beispiel *Staphylococcus aureus* (DSMZ 18587) und *Staphylococcus aureus* (DSMZ 20232) nach Standardprotokollen kultiviert. Dafür wurde TSY (Trypticase Soy Yeast Extract Medium) verwendet.

Die Aufarbeitung der Targetstämme und der Co-Aggregationsassay erfolgte wie im Beispiel 2 beschrieben.

Die Versuche ergaben, dass auch weitere Keime der gleichen Art und Gattung zur Bindung bzw. Aggregation befähigt sind durch die erfindungsgemäßen *Lactobacillus*-Stämme. Beispielhaft für die erfindungsgemäßen Mikroorganismen sind in Figur 10 die Ergebnisse des Co-Aggregationsassays für Lactobacillus DSM 25906 und DSM 25907 mit *Staphylococcus aureus* DSMZ 18587 und DSMZ 20232 dargestellt.

**Beispiel 6**

Biofilmhemmung

Zur Identifizierung und Selektion von erfindungsgemäßen Mikroorganismen wurden aus einer *Lactobacillus*-Datenbank verschiedene Stämme in einem Screeningverfahren hinsichtlich der biofilm-verhindernden Eigenschaften der pathogene Hautkeime (im Folgenden auch "Targetkeime") im Mikrotiterplatten-Maßstab geprüft. Die Stärke der biofilmverhindernen Eigenschaften konnten quantitativ gegenüber der unbeeinflussten Biofilmbildung der Targetkeime in Relation gebracht und analysiert werden, was schließlich zu den identifizierten beispielhaften erfindungsgemäßen Mikroorganismen (*Lactobacillus*) führte.

Zur Untersuchung ihrer Wirkung auf die Biofilmbildung der pathogenen Targetkeime *Staphylococcus aureus* (DSM 18587) oder *Pseudomonas aeruginosa* (DSM 22644) wurden die *Lactobacillen*-Stämme bzw. deren Fraktionen direkt zu dem Targetkeim zu Beginn der Biofilmausbildung zugegeben und für bis zu 6 h inkubiert. Nach der Entfernung der nicht gebundenen Zellen und 2 x Waschen der Biofilme mit PBS erfolgte die Quantifizierung der Biofilme durch Messung der optischen Dichte nach Kristallviolettfärbung des gesamten Ansatzes.

Zur Durchführung dieser Versuche wurden die Targetstämme, wie weiter oben beschrieben, nach Standard-Protokoll kultiviert, sowie die *Lactobacillus*-Stämme in MRS-Medium anaerob kultiviert.

Zur Aufarbeitung der *Lactobacillus*-Stämme wurden diese nach Kultivierung 2 Mal mit PBS gewaschen und in PBS aufgenommen.

Ein Teil der *Lactobacillus*-Stämme nach PBS-Waschung wurde hitze-inaktiviert durch Pasteurisieren bei 70°C für 30min.

Des Weiteren wurde Teil der hitze-inaktivierten *Lactobacillus*-Stämme nach PBS-Waschung wurde verwendet für die Gewinnung der Überstande. Dies erfolgte durch Zentrifugation der Suspension und anschließender Verwendung der Überstände.

Zur Aufarbeitung der Targetstämme wurden diese bis zum Erreichen der mittleren exponentiellen Wachstumsphase kultiviert, geerntet und auf die $OD600_{nm(ml-1)}$=3,5 eingestellt, 2 Mal mit PBS gewaschen und in PBS aufgenommen.

Zur Durchführung des Biofilmbildungs-Assays wurden die *Lactobacillus*-Stämme direkt zu dem Targetkeim zu Beginn der Biofilmausbildung zugegeben und für 6 h, anaerob, bei 37 °C inkubiert. Parallel erfolgten Kontrollansätze ohne Zugabe der *Lactobacillus*-Suspension. Nach der Entfernung der planktonischen Zellen und Waschen der Biofilme erfolgte die Quantifizierung der Biofilme durch Kristallviolettfärbung (0,1 %, Merck) des gebundenen Targetkeimes mit bzw. ohne *Lactobacillus* bzw. *Lactobacillus-Fraktion.* Hierzu wurden die gebundenen Zellen der jeweiligen Ansätze nach Kristallviolettfärbung mittels Essigsäure abgelöst und in Suspension gebracht und bei einer optischen Dichte von 590 nm vermessen. Die Reduktion der optischen Dichte bei 590nm im Vergleich zur Kontrolle des Targetkeims ohne *Lactobacillen* korrelierte dabei mit der Stärke der Biofilmverhinderung. Diese Reduktion wird als prozentuale Hemmung der Biofilmbildung dargestellt in Relation zu Targetkeim ohne *Lactobacillen.* Beispielhaft für die erfindungsgemäßen Mikroorganismen ist in Figur 11 das Ergebnis der Biofilmhemmung von *Staphylococcus aureus* durch *Lactobacillus* DSM 25914 und DSM 25915 und von *Pseudomonas aeruginosa* durch DSM 25912 und DSM 25913 in Fig. 12 dargestellt. Die Versuche ergaben, dass es zu einer 56-93% Hemmung der Biofilmbildung durch die erfindungsgemäßen *Lactobacillus*-Stämme, mit und ohne Inaktivierung der metabolischen Aktivitäten der *Lactobacillus*-Stämme bzw. deren Fraktionen von *Staphylococcus aureus* bzw. *Pseudomonas aeruginosa* führt.

Die Experimente und Beispiele zeigen nur eine Auswahl an bevorzugten Mikroorganismen, wobei die gemeinsamen vorteilhaften Eigenschaften für alle der bevorzugten Mikroorganismen experimentell bestätigt wurden. Der Fachmann erfährt aus den Beispielen und den Figuren, wie er die Erfindung nachzuarbeiten hat und z. B. die gemeinsamen vorteilhaften Eigenschaften der bevorzugten Mikroorganismen reproduziert.

**Referenzen:**

Davies CE, Hill KE, Wilson MJ, Stephens P, Hill CM, Harding KG and Thomas DW (2004) Use of 16S ribosomal DNA PCR and denaturing gradient gel electrophoresis for analysis of the microfloras of healing and nonhealing chronic venous leg ulcurs. J Clin Microbiol 42, 3549-57.

Fux, CA, Costerton, JW, Stewart, PS, Stoodley P (2005) Survival strategies of infectious biofilms. Trends in Microbiology 13, 34-40.

James TJ, Hughes MA, Cherry GW, Taylor RP. Evidence of oxidative stress in chronic venous ulcers. Wound Repair Regen 2003; 11(3): 172-6.

James GA, Swogger E, Wolcott R, Pulcini E, Secor P, Sestrich J, Costerton JW, Stewart PS (2008) Biofilms in chronic wounds. Wound Repair Regen 16, 37-44.

Kirketerp-Moller K, Jensen PØ, Fazli M, Madsen KG, Pedersen J, Moser, C, Tolker-Nielsen, T, Hoiby N, Givskov M, and Bjarnsholt T (2008) Distribution, Organization, and Ecology of Bacteria in Chronic Wounds. J Clin Microbiol 46, 2717-2722.

Mast BA, Schultz GS. Interactions of cytokines, growth factors, and proteases in acute and chronic wounds. Wound Repair Regen 1996; 4(4): 411-20.

Moseley R, Hilton JR, Waddington RJ, Harding KG, Stephens P, Thomas DW. Comparison of oxidative stress biomarker profiles between acute and chronic wound environments. Wound Repair Regen 2004; 12(4): 419-29.

Sheldon AT (2005) Antiseptic "resistance": real or perceived threat? Clin. Infect. Dis. 40, 1650-1656.

Julia Bidder, MRSA, http://www.focus.de/gesundheit/arzt-klinik/klinik/tid-9019/mrsa-krank-durch-die-klinik_aid_262385.html, 26.02.2010

Bingham R. J.; Rudino-Pinera E.; Meenan N. A. G.; Schwarz-Linek U.; Turkenburg J. P.; Hook M.; Garman E. F.; Potts J. R. Proc. Natl. Acad. Sci. U.S.A. 2008, 105, 12254-12258. O'Neill E.; Pozzi C.; Houston P.; Humphreys H.; Robinson D. A.; Loughman A.; Foster T. J.; O'Gara J. P. J. Bacteriol. 2008, 190, 3835-3850.

de Man et al., (1960) "A medium for the Cultivation of Lactobacilli", J. Appl. Bact. 23(130-135)

## Patentansprüche

1. Milchsäurebakterium oder Zelllysat, Derivat, Mutante oder Kombination davon, wobei der Mikroorganismus, oder Zelllysat, Derivat, Mutante oder Kombination davon mit mindestens einem pathogenen Mikroorganismus koaggregieren kann, wobei der pathogene Mikroorganismus ausgewählt ist aus der Gruppe umfassend *Staphylococcus aureus* oder *Pseudomonas aeruginosa* und wobei das Milchsäurebakterium ausgewählt ist aus der Gruppe umfassend folgende bei der Deutschen Sammlung für Mikroorganismen und Zellkulturen hinterlegten als DSM 25906, DSM 25907, DSM 25908, DSM 25909, DSM 25910, DSM 25911, DSM 25912, DSM 25913, DSM 25914 und DSMZ 25915 nummerierten Mikroorganismen.

2. Milchsäurebakterium oder Zelllysat, Derivat, Mutante oder Kombination davon nach Anspruch 1 oder 2, wobei die Fähigkeit zur Koaggregation des zumindest einen pathogenen Mikroorganismus auch nach einer biologischen, chemischen oder physikalischen Behandlung besteht und/oder wobei die Fähigkeit zur Koaggregation des zumindest einen pathogenen Mikroorganismus bei einem pH-Wert von zwischen ca. 3 bis ca. 8 besteht.

3. Milchsäurebakterium oder Zelllysat, Derivat, Mutante oder Kombination davon nach einem oder mehreren der vorherigen Ansprüche, wobei das Milchsäurebakterium oder Zelllysat, Derivat, Mutante oder Kombination davon die Fähigkeit zur Hemmung einer Bildung eines Biofilms des zumindest einen pathogenen Mikroorganismus besitzt.

4. Zusammensetzung umfassend mindestens ein Milchsäurebakterium oder Zelllysat, Derivat, Mutante oder Kombination davon nach einem oder mehreren der vorherigen Ansprüche, wobei die Zusammensetzung einen Träger oder Exzipienten, ausgewählt aus der Gruppe umfassend kosmetisch verträgliche Träger oder Exzipienten, pharmazeutisch verträgliche Träger oder Exzipienten oder dermatologisch verträgliche Träger oder Exzipienten umfasst und/oder wobei die Zusammensetzung als Puder, in Stiftform, als Aerosolspray, Pumpspray, Creme, Dispersion, Emulsion, Schaum, Salbe, Spray, Aerosol, Pulver, Stift, Tücher, Lotion, Suspension, Lösung, Gel oder auf einem Substrat vorliegt und/oder
wobei die Zusammensetzung in fester, flüssiger, viskoser Form oder als Aerosol und/oder als Hautcreme, Hautwaschlotion oder Hautsalbe vorliegt und/oder
wobei die Zusammensetzung Probiotika, Antiseptika oder andere antibakteriell wirksame Substanzen umfasst.

5. Zusammensetzung nach dem vorherigen Anspruch, wobei es sich um eine pharmazeutische, tiermedizinische, kosmetische oder Nahrungsmittelzusammensetzung handelt.

6. Zusammensetzung nach Anspruch 4 oder 5, weiterhin umfassend zumindest einen Stoff, ausgewählt aus den Gruppen

    a. Wirkstoffe, die den Zustand der Haut positiv beeinflussen, insbesondere Wirkstoffe zur positiven Beeinflussung der Altershaut, insbesondere in Kombination mit Biochinone, insbesondere Ubichinon Q10, Kreatin, Kreatinin, Carnitin, Biotin, Isoflavon, Cardiolipin, Liponsäure, Anti Freezing Proteine, Arctiin, Hopfen- und Hopfen-Malz-Extrakte,
    b. fördernde Mitteln zur Restrukturierung des Bindegewebes, insbesondere Isoflavonoide,
    c. Wirkstoffen zur Unterstützung der Hautfunktionen bei trockener Haut, insbesondere Vitamin C, Biotin, Carnitin, Kreatin, Propionsäure, Grüntee-Extrakte, Eucalyptusöl, Harnstoff und Mineralsalze, insbesondere NaCl, Meeresmineralien sowie Osmolyte,
    d. Wirkstoffen zur Linderung und/oder positiven Beeinflussung von irritativen Hautzuständen, insbesondere Sericoside, verschiedene Extrakte des Süssholzes, Licochalcone, insbesondere Licochalcone A, Silymarin, Silyphos und/oder Dexpanthenol.

7. Zusammensetzung nach einem oder mehreren der Ansprüche 4 bis 6, wobei das Milchsäurebakterium inaktiviert, lebend oder nicht-lebend in der Zusammensetzung vorliegt und/oder
wobei das Milchsäurebakterium in verkapselter, sprühgetrockneter und/oder lyophilisierter Form vorliegt und/oder.
wobei das Milchsäurebakterium in Form eines Zelllysates vorliegt und/oder
wobei das Milchsäurebakterium in einer Menge mit einem Masseanteil von 0,001 Gewichtsprozent bis 10 Gewichtsprozent, bevorzugt bis 0,005 Gewichtsprozent bis 5 Gewichtsprozent, besonders bevorzugt 0,01 Gewichtsprozent bis 3 Gewichtsprozent vorliegt.

8. Verwendung des Milchsäurebakteriums nach einem oder mehreren der Ansprüche 1 bis 3 oder der Zusammensetzung nach einem oder mehreren der Ansprüche 4 bis 7 zur Herstellung eines Arzneimittels, Medizinproduktes oder Kosmetikums zur Behandlung oder Prophylaxe von Hauterkrankungen, insbesondere Staphylococcal scalded skin syndrome, Impetigo contagiosa, Folliculitis superficialis, Impetiginisation, Hautabszesse, Furunkel, Karbunkel, Eiterbeule, Phlegmone, trockene Haut, juckende Haut, gerötete Haut, irritierte Haut, stark fettende Haut, Akne, diabetischer Fuß, Dekubitus, Neurodermitis, Akute Lymphadenitis, Pilonidalzysten, Pilonidalfistel, Pilonidalsinus, Steißbeinfistel, Steißbeinzyste, lokale Infektionen der Haut und der Unterhaut, Pyodermie, Dermatitis purulenta, Dermatitis septica, Dermatitis suppurativa, Dermatitis und Ekzeme, atopisches Ekzem, seborrhoisches Ekzem, Windeldermatitis, allergische Kontaktdermatitis, seborrhoisches Dermatitis, exfoliative Dermatitis, toxische Kontaktdermatitis, Lichen simplex chronicus, Prurigo, Pruritus sowie sonstige Dermatitis, papulosquamöse Hautkrankheiten, Psoriasis, Parapsoriasis, Krankheiten der Hautanhangsgebilde, narbige Alopezie, Folliculitis decalvans, sowie sonstige Erkrankungen der Haut und Unterhaut, Ulcus cruris, Hautverletzungen, Schürfwunden, Wunden, auch nach Unfällen oder Operationen.

9. Verwendung des Milchsäurebakteriums nach einem oder mehreren der Ansprüche 1 bis 3 oder der Zusammensetzung nach einem oder mehreren der Ansprüche 4 bis 7 zur Herstellung eines Reinigungsmittels oder Desinfektionsmittels für die Behandlung von Oberflächen.

10. Verwendung des Milchsäurebakteriums nach einem oder mehreren der Ansprüche 1 bis 3 oder der Zusammensetzung nach einem oder mehreren der Ansprüche 4 bis 7 zur Herstellung eines Produktes, das im Bereich der Körperhygiene, Medizinprodukte und Prophylaxe eingesetzt wird.

11. Verwendung des Milchsäurebakteriums nach einem oder mehreren der Ansprüche 1 bis 3 oder der Zusammensetzung nach einem oder mehreren der Ansprüche 4 bis 7 zur Herstellung eines spezifisch wirkendes antimikrobielles Additiv für die lokale Behandlung von Hautinfektionen und die Beschleunigung der Heilung von chronischen Wunden.

12. Verwendung nach einem oder mehreren der vorherigen Ansprüche, wobei die Zusammensetzung prophylaktisch oder kurativ verwendet wird.

13. Verwendung nach einem oder mehreren der vorherigen Ansprüche, wobei die Zusammensetzung topisch appliziert wird.

14. Kit zur Hygienebehandlung, umfassend Milchsäurebakterien nach Anspruch 1 bis 3 oder die Zusammensetzung nach den Ansprüche 4 bis 7 und körperhygienische Vorrichtungen oder Geräte, Spülungen und/oder Pasten.

**Claims**

1. A lactic acid bacterium or cell lysate, derivative, mutant or combination thereof, wherein the microorganism, or cell lysate, derivative, mutant or combination thereof can coaggregate with at least one pathogenic microorganism, wherein the pathogenic microorganism is selected from the group comprising *Staphylococcus aureus* or *Pseudomonas aeruginosa* and wherein the lactic acid bacterium is selected from the group comprising the following microorganisms deposited with the German Collection of Microorganisms and Cell Cultures under number DSM 25906, DSM 25907, DSM 25908, DSM 25909, DSM 25910, DSM 25911, DSM 25912, DSM 25913, DSM 25914 and DSMZ 25915.

2. The lactic acid bacterium or cell lysate, derivative, mutant or combination thereof according to Claim 1 or 2, wherein the coaggregation ability of the at least one pathogenic microorganism exists even after a biological, chemical or physical treatment and/or
wherein the coaggregation ability of the at least one pathogenic microorganism exists at a pH between approximately 3 and approximately 8.

3. The lactic acid bacterium or cell lysate, derivative, mutant or combination thereof according to one or more of the preceding claims, wherein the lactic acid bacterium or cell lysate, derivative, mutant or combination thereof has the ability to inhibit a formation of a biofilm of the at least one pathogenic microorganism.

4. A composition comprising at least one lactic acid bacterium or cell lysate, derivative, mutant or combination thereof according to one or more of the preceding claims, wherein the composition comprises a carrier or excipient selected from the group comprising cosmetically acceptable carriers or excipients, pharmaceutically acceptable carriers or excipients or dermatologically acceptable carriers or excipients and/or wherein the composition is in the form of a powder, stick, aerosol spray, pump spray, cream, dispersion, emulsion, foam, ointment, spray, aerosol, powder, stick, cloths, lotion, suspension, solution, gel or on a substrate and/or
wherein the composition is present in solid, liquid, viscous form or as an aerosol and/or as a skin cream, skin washing lotion, or skin ointment
and/or
wherein the composition comprises probiotics, antiseptics or other antibacterially active substances.

5. The composition according to the preceding claim, wherein said composition is a pharmaceutical, veterinary, cosmetic or food composition.

6. The composition according to Claim 4 or 5, moreover comprising at least one substance, selected from the groups

    a. active ingredients which have a positive influence on the condition of the skin, in particular active ingredients for having a positive influence on elderly skin, in particular in combination with bioquinones, in particular ubiquinone Q10, creatine, creatinine, carnitine, biotin, isoflavone, cardiolipin, lipoic acid, antifreezing proteins, arctiin, hops and hop-malt extracts,
    b. promoting agents for restructuring the connective tissue, in particular isoflavonoids,
    c. active ingredients for supporting skin functions in the case of dry skin, in particular vitamin C, biotin, carnitine, creatine, propionic acid, green tea extracts, eucalyptus oil, urea and mineral salts, in particular NaCl, marine minerals as well as osmolytes,
    d. active ingredients for relieving and/or having a positive influence on irritated skin conditions, in particular sericosides, various extracts of licorice, licochalcones, in particular licochalcone A, silymarin, silyphos and/or dexpanthenol.

7. The composition according to one or more of Claims 4 to 6, wherein the lactic acid bacterium is present in inactivated, viable or nonviable form in the composition and/or
wherein the lactic acid bacterium is present in encapsulated, spray-dried and/or lyophilized form and/or
wherein the lactic acid bacterium is in the form of a cell lysate and/or
wherein the lactic acid bacterium is present in an amount with a mass portion of 0.001 percent by weight to 10 percent by weight, preferably 0.005 percent by weight to 5 percent by weight, in particular preferably 0.01 percent by weight to 3 percent by weight.

8. Use of the lactic acid bacterium according to one or more of Claims 1 to 3 or of the composition according to one or more of Claims 4 to 7 for producing a drug, medical product or cosmetic for the treatment or prophylaxis of skin diseases, in particular staphylococcal scalded skin syndrome, impetigo contagiosa, folliculitis superficialis, impetiginisation, skin abscesses, furuncles, carbuncles, abscesses, phlegmons, dry skin, itchy skin, reddened skin, irritated skin, extremely oily skin, acne, diabetic foot, decubital ulcers, neurodermatitis, acute lymphadenitis, pilonidal cysts, pilonidal fistula, pilonidal sinus, coccygeal fistula, coccygeal cyst, local infections of the skin and the subcutaneous tissue, pyoderma, purulent dermatitis, septic dermatitis, suppurative dermatitis, dermatitis and eczema, atopic eczema, seborrheic eczema, exfoliative dermatitis, allergic contact dermatitis, seborrheic dermatitis, exfoliative dermatitis, toxic contact dermatitis, chronic lichen simplex, prurigo, pruritus and other forms of dermatitis, papulosquamous skin diseases, psoriasis, parapsoriasis, diseases of the integumentary appendages, alopecia with scarring, folliculitis decalvans, as well as other diseases of the skin and subcutaneous tissue, crural ulcer, skin injuries, abrasions, wounds, including those after accidents or surgery.

9. Use of the lactic acid bacterium according to one or more of Claims 1 to 3 or of the composition according to one or more of Claims 4 to 7 for producing a cleaning agent or disinfectant for the treatment of surfaces.

10. The use of the lactic acid bacterium according to one or more of Claims 1 to 3 or of the composition according to one or more of Claims 4 to 7 for producing a product which is used in the field of body hygiene, medical products and prophylaxis.

11. The use of the lactic acid bacterium according to one or more of Claims 1 to 3 or of the composition according to one or more of Claims 4 to 7 for producing a specifically acting antimicrobial additive for the local treatment of skin diseases and for accelerating the healing of chronic wounds.

12. The use according to one or more of the preceding claims, wherein the composition is used prophylactically or curatively.

13. The use according to one or more of the preceding claims, wherein the composition is applied topically.

14. Kit for hygiene treatment comprising lactic acid bacteria according to Claims 1 to 3 or the composition according to Claims 4 to 7, and physical hygiene devices or equipment, rinses and/or pastes.


**Revendications**

1. Bactérie d'acide lactique ou lysat cellulaire, dérivé, mutants ou leur combinaison, dans laquelle le micro-organisme ou le lysat cellulaire, dérivé, mutants ou leur combinaison peuvent être co-agrégés avec au moins un micro-organisme pathogène, dans laquelle le micro-organisme pathogène est choisi dans le groupe comprenant Staphylococcus aureus ou Pseudomonas aeruginosa et dans laquelle la bactérie d'acide lactique est choisie dans le groupe comprenant les micro-organismes enregistrés dans la Deutschen Sammlung für Mikroorganismen und Zellkulturen (collection allemande de micro-organismes et cultures cellulaires) sous les numéros : DSM 25906, DSM 25907, DSM 25908, DSM 25909, DSM 25910, DSM 25911, DSM 25912, DSM 25913, DSM 25914 et DSMZ 25915.

2. Bactérie d'acide lactique ou lysat cellulaire, dérivé, mutants ou leur combinaison selon la revendication 1 ou 2, dans laquelle la capacité de co-agrégation de l'au moins un micro-organisme pathogène existe également après un traitement biologique, chimique ou physique, et/ou
dans laquelle la capacité de co-agrégation de l'au moins un micro-organisme pathogène existe à un pH entre environ 3 à environ 8.

3. Bactérie d'acide lactique ou lysat cellulaire, dérivé, mutants ou leur combinaison selon une ou plusieurs des revendications précédentes, dans laquelle la bactérie d'acide lactique ou lysat cellulaire, dérivé, mutants ou leur combinaison possèdent la capacité d'inhiber une formation d'un biofilm de l'au moins un micro-organisme pathogène.

4. Composition comprenant au moins une bactérie d'acide lactique ou lysat cellulaire, dérivé, mutants ou leur combinaison selon une ou plusieurs des revendications précédentes, dans laquelle la composition comprend un véhicule ou des excipients, choisis dans le groupe comprenant des véhicules ou excipients acceptables d'un point de vue cosmétique, des véhicules ou excipients pharmaceutiquement acceptables ou des véhicules ou excipients dermatologiquement acceptables et/ou dans laquelle la composition se présente sous forme de poudre, de bâton, d'aérosol, de vaporisateur, de crème, de dispersion, d'émulsion, de mousse, de pommade, de spray, d'aérosol, de poudre, de bâton, de lingette, de lotion, de suspension, de solution, de gel ou d'un substrat et/ou dans laquelle la composition se présente sous forme solide, fluide, visqueuse ou en aérosol et/ou sous forme de crème pour la peau, lotion de toilette ou pommade pour la peau et/ou dans laquelle la composition comprend des probiotiques, des antiseptiques ou autres substances actives comme antibactérien.

5. Composition selon la revendication précédente, dans laquelle il s'agit d'une composition pharmaceutique, de médecine vétérinaire, cosmétique ou alimentaire.

6. Composition selon la revendication 4 ou 5, comprenant en outre au moins une substance choisie parmi les groupes de

   a. principes actifs qui exercent une action positive sur l'état de la peau, notamment des principes actifs destinés à agir de façon positive sur la peau âgée, notamment en combinaison avec des bioquinones, notamment l'ubiquinone Q10, la créatine, la créatinine, la carnitine, la biotine, l'isoflavone, la cardiolipine, l'acide lipoïque, les protéines anti-gel, l'arctiine, des extraits de houblon et de malt,
   b. des agents favorisation la restructuration du tissu conjonctif, notamment des isoflavonoïdes,

c. des principes actifs pour soutenir les fonctions cutanées en cas de sécheresse cutanée, notamment la vitamine C, la biotine, la carnitine, la créatine, l'acide propionique, des extraits de thé vert, l'huile d'eucalyptus, l'urée et des sels minéraux, notamment NaCl, des minéraux marins ainsi que des osmolytes,

d. des principes actifs pour calmer et/ou exercer un effet positif sur les états cutanés irrités, notamment les séricosides, différents extraits de réglisse, les licochalcones, notamment les licochalcones A, la silymarine, le silyphos et/ou le dexpanthénol.

**7.** Composition selon une ou plusieurs des revendications 4 à 6, dans laquelle la bactérie d'acide lactique se présente inactivée, vivante ou non vivante dans la composition et/ou dans laquelle la bactérie d'acide lactique se présente sous forme encapsulée, séchée par pulvérisation et/ou lyophilisée, et/ou

dans laquelle la bactérie d'acide lactique se présente sous la forme d'un lysat cellulaire et/ou

dans laquelle la bactérie d'acide lactique se présente en une quantité présentant un rapport massique de 0,001 % en poids à 10 % en poids, de préférence jusqu'à 0,005 % en poids à 5 % en poids, de manière particulièrement préférée, de 0,01 % en poids à 3 % en poids.

**8.** Utilisation de la bactérie d'acide lactique selon une ou plusieurs des revendications 1 à 3 ou de la composition selon une ou plusieurs des revendications 4 à 7, pour la production d'un médicament, d'un produit médicamenteux ou d'un cosmétique pour le traitement ou la prophylaxie des maladies de peau, notamment du syndrome de peau squameuse à staphylocoques, l'impétigo, la folliculite superficielle, l'impétiginisation, les abcès cutanés, les furoncles, les anthrax, les pustules, les phlegmons, la sécheresse cutanée, les démangeaisons cutanées, les rougeurs cutanées, les irritations cutanées, les peaux très grasses, l'acné, le pied diabétique, les escarres de décubitus, la neurodermite, la lymphadénite aiguë, les kystes pilonidaux, les fistules pilonidales, le sinus pilonidal, les fistules coccygiennes, les kystes coccygiens, les infections locales de la peau et des tissus sous-cutanés, la pyodermie, la dermatite purulente, la dermatite septique, la dermatite suppurative, la dermatite et l'eczéma, l'eczéma atopique, l'eczéma séborrhéique, l'érythème fessier, la dermatite de contact allergique, la dermatite séborrhéique, la dermatite exfoliative, la dermatite de contact toxique, le lichen simplex chronique, le prurigo, le prurit et autres dermatites, les maladies cutanées papulosquameuses, le psoriasis, le parapsoriasis, les maladies des annexes cutanées, l'alopécie cicatricielle, la folliculite décalvante, ainsi que d'autres maladies de la peau et des tissus sous-cutanés, l'ulcère crural, des lésions cutanées, des abrasions, des plaies, également consécutives à des accidents ou opérations.

**9.** Utilisation de la bactérie d'acide lactique selon une ou plusieurs des revendications 1 à 3 ou de la composition selon une ou plusieurs des revendications 4 à 7 pour la production d'un agent nettoyant ou d'un agent désinfectant pour le traitement des surfaces.

**10.** Utilisation de la bactérie d'acide lactique selon une ou plusieurs des revendications 1 à 3 ou de la composition selon une ou plusieurs des revendications 4 à 7 pour la production d'un produit qui est utilisé dans le domaine de l'hygiène corporelle, des produits médicaux et de la prophylaxie.

**11.** Utilisation de la bactérie d'acide lactique selon une ou plusieurs des revendications 1 à 3 ou de la composition selon une ou plusieurs des revendications 4 à 7 pour la production d'un additif antimicrobien à action spécifique pour le traitement local des infections cutanées et l'accélération de la cicatrisation des plaies chroniques.

**12.** Utilisation selon une ou plusieurs des revendications précédentes, dans laquelle la composition est utilisée sur le plan prophylactique ou curatif.

**13.** Utilisation selon une ou plusieurs des revendications précédentes, dans laquelle la composition est appliquée par voie topique.

**14.** Kit de traitement hygiénique, comprenant des bactéries d'acide lactique selon la revendication 1 à 3 ou la composition selon les revendications 4 à 7 et des dispositifs ou appareils d'hygiène corporelle, des rinçages et/ou des pâtes.

**Figur 1**

Figur 2

**Figur 3**

**Figur 4**

|  | Co-Aggregation | Lactobacillus separat | Pseudomonas aeruginosa separat |
|---|---|---|---|
| DSM 25909 | | | |
| DSM 25910 | | | |
| DSM 25911 | | | |

**Figur 5**

Co-Aggregation
*Lactobacillus + Staphylococcus aureus*

DSM 25906  DSM 25907  DSM 25908

*Staphylococcus aureus*
separat

*Lactobacillus*
*separat*

**Figur 6**

**Figur 7**

**Figur 8**

**Figur 9**

**Figur 10**

**Figur 11**

Figur 12

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2010130563 A
- US 6790434 B
- WO 9725865 A
- WO 2008064893 A1
- WO 2005117921 A
- US 6187323 B, Aiache
- EP 2133414 A1

### In der Beschreibung aufgeführte Nicht-Patentliteratur

- **SOLEIMANI et al.** Antagonistic activity of probiotic lactobacilli against Staphylococcus aureus isolated from bovine mastitis. *African Journal of Microbiology Research,* 18. Oktober 2010, vol. 4 (20
- **DANIELS ; KNIE.** *JDDG,* 2007, vol. 5, 367-383
- *JDDG,* 2007, vol. 5, 367-383
- **SCHLEIFER et al.** *System. Appl. Microb.,* 1995, vol. 18, 461-467
- **LUDWIQ et al.** *System. Appl. Microb.,* 1992, vol. 15, 487-501
- **DAVIES CE ; HILL KE ; WILSON MJ ; STEPHENS P ; HILL CM ; HARDING KG ; THOMAS DW.** Use of 16S ribosomal DNA PCR and denaturing gradient gel electrophoresis for analysis of the microfloras of healing and nonhealing chronic venous leg ulcurs. *J Clin Microbiol,* 2004, vol. 42, 3549-57
- **FUX, CA ; COSTERTON, JW ; STEWART, PS ; STOODLEY P.** Survival strategies of infectious biofilms. *Trends in Microbiology,* 2005, vol. 13, 34-40
- **JAMES TJ ; HUGHES MA ; CHERRY GW ; TAYLOR RP.** Evidence of oxidative stress in chronic venous ulcers. *Wound Repair Regen,* 2003, vol. 11 (3), 172-6
- **JAMES GA ; SWOGGER E ; WOLCOTT R ; PULCINI E ; SECOR P ; SESTRICH J ; COSTERTON JW ; STEWART PS.** Biofilms in chronic wounds. *Wound Repair Regen,* 2008, vol. 16, 37-44
- **KIRKETERP-MOLLER K ; JENSEN PØ ; FAZLI M ; MADSEN KG ; PEDERSEN J ; MOSER, C ; TOLKER-NIELSEN, T ; HOIBY N ; GIVSKOV M ; BJARNSHOLT T.** Distribution, Organization, and Ecology of Bacteria in Chronic Wounds. *J Clin Microbiol,* 2008, vol. 46, 2717-2722
- **MAST BA ; SCHULTZ GS.** Interactions of cytokines, growth factors, and proteases in acute and chronic wounds. *Wound Repair Regen,* 1996, vol. 4 (4), 411-20
- **MOSELEY R ; HILTON JR ; WADDINGTON RJ ; HARDING KG ; STEPHENS P ; THOMAS DW.** Comparison of oxidative stress biomarker profiles between acute and chronic wound environments. *Wound Repair Regen,* 2004, vol. 12 (4), 419-29
- **SHELDON AT.** Antiseptic ''resistance'': real or perceived threat?. *Clin. Infect. Dis.,* 2005, vol. 40, 1650-1656
- **JULIA BIDDER.** *MRSA,* 26. Februar 2010, http://www.focus.de/gesundheit/arzt-klinik/klinik/tid-9019/mrsa-krank-durch-die-klinik_aid_262385.html
- **BINGHAM R. J. ; RUDINO-PINERA E. ; MEENAN N. A. G. ; SCHWARZ-LINEK U. ; TURKENBURG J. P. ; HOOK M. ; GARMAN E. F. ; POTTS J. R.** *Proc. Natl. Acad. Sci. U.S.A.,* 2008, vol. 105, 12254-12258
- **O'NEILL E. ; POZZI C. ; HOUSTON P. ; HUMPHREYS H. ; ROBINSON D. A. ; LOUGHMAN A. ; FOSTER T. J. ; O'GARA J. P.** *J. Bacteriol.,* 2008, vol. 190, 3835-3850
- **DE MAN et al.** A medium for the Cultivation of Lactobacilli. *J. Appl. Bact.,* 1960, vol. 23, 130-135